(19) **Europäisches Patentamt**
**European Patent Office**
**Office européen des brevets**

(11) **EP 2 189 919 A1**

(12) **EUROPEAN PATENT APPLICATION**

(43) Date of publication:
26.05.2010 Bulletin 2010/21

(51) Int Cl.:
*G06F 19/00* (2006.01)    *A61K 39/145* (2006.01)

(21) Application number: 08020435.7

(22) Date of filing: 25.11.2008

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Max-Planck-Gesellschaft zur Förderung der Wissenschaften e.V.
80539 München (DE)**

(72) Inventors:
• **McHardy, Alice Carolyn, Dr.
66111 Saarbrücken (DE)**
• **Steinbrück, Lars
66111 Saarbrücken (DE)**

(74) Representative: **Grünecker, Kinkeldey, Stockmair & Schwanhäusser
Anwaltssozietät
Leopoldstrasse 4
80802 München (DE)**

(54) **Method and system for building a phylogeny from genetic sequences and using the same for recommendation of vaccine strain candidates for the influenza virus**

(57)    A computer-implemented method and a computer system for identifying a phylogenetic tree from a plurality of biological sequences is provided. Each biological sequence is associated with a sampling date. First, the plurality of biological sequences is aligned and a distance matrix is obtained. Then, a subset of these sequences without any duplicated sequences is selected and a directed graph representation of the subset of biological sequences is generated based on the associated sampling dates. Then, a minimum spanning tree is computed from the weighted directed graph representation. Then, in an iterative procedure, the sequences of unsampled evolutionary intermediates are inferred from mutation patterns that reflect the difference in sequence between the nodes in the minimum spanning tree. The new sequences are added with associated time stamps to the sequence set. Then, sets of identical sequences are removed. Then, an optimum branching is recomputed. This step is repeated until no new intermediates are found. In the final step, the sequences that have been set aside in the inititalizing step are added to the plurality of sequences derived in the update step. From this plurality of sequences an optimum branching is computed and identified as the phylogenetic tree. Amino acid changes repeatedly occurring on the internal branches of the obtained tree can be used to identify sequences and associated viral isolates suitable as vaccine strains for the following influenza season.

FIG. 1

**Description**

**BACKGROUND OF THE INVENTION**

**1. Field of the Invention**

The invention relates to the construction of phylogenetic trees and more particular to their application to identify vaccine strain isolates for the influenza virus.

**2. Description of the Related Art**

Methods for constructing phylogenetic trees are of central importance in the study of viral diseases. Phylogenies indicate the importance of individual sequence sites and mutation events in evolution, allow identification of fast evolving sites and sites under positive or purifying selection, and give insight into the timing and direction of mutation events. Phylogenies and the mutation events mapping onto their internal edges are studied in the process of vaccine strain selection for seasonal influenza, to monitor viral geographic spread, the appearance and prevalence of drug-resistant viral variants, the detection of host transfer events for viruses from different host reservoirs and to identify the emergence of human transmissible influenza strains with the potential to cause a pandemic.

Conventionally, phylogenetic techniques search for tree topologies under a given optimality criterion with tree rearrangement operations such as nearest-neighbor interchanges, subtree pruning and regrafting or tree bisection and reconnection. This is allows the construction of phylogenies from sets of sequences at arbitrary evolutionary distances, but becomes computationally very demanding for large sequence sets.

For instance, S. Khumar et al. "MEGA3: Integrated software for Molecular Evolutionary Genetics Analysis and sequence alignment", BRIEFINGS IN BIOINFORMATICS, VOL 5, NO 2, pages 150-163, describes the Molecular Evolutionary Genetics Analysis (MEGA) software, with its focus on facilitating the exploration and analysis of the DNA and protein sequence variation from an evolutionary perspective. The third major release, MEGA3, contains facilities for automatic and manual sequence alignment, web-based mining of databases, inference of the phylogenetic trees, estimation of evolutionary distances and testing evolutionary hypotheses. Phylogenetic trees can be constructed with distance-matrix methods such as neighbor-joining or UPGMA (Unweighted Pair Group Method with Arithmetic mean), based on genetic distances inferred from multiple sequence alignments. UPGMA is known to not perform well when evolution is non-clocklike, i.e. when the rate of evolution varies along different branches. Neighbor-joining phylogenies are often used as a starting point for methods such as described below, to further improve the tree under a given optimality criterion.

More advanced methods use optimality criteria such as maximum parsimony (least number of overall mutations), maximum likelihood or Bayesian inference under an explicit model of sequence evolution. Available software for inference of phylogenies under these criteria include PAUP (Swofford, D.L. "PAUP: Phylogenetic Analysis Using Parsimony (*and Other Methods)", Version 4.0d64 ed, Sinauer Associates, Inc. Publishers, Sunderland, MA.), GARLI (Zwickl, D. J. "Genetic algorithm approaches for the phylogenetic analysis of large biological sequence datasets under the maximum likelihood criterion". Ph.D. dissertation, The University of Texas at Austin., PhyML (Guindon and Olivier Gascuel "A simple, fast and accurate method to estimate large phylogenies by maximum-likelihood", SYSTEM BIOLOGY VOL 52, pages 696-704) and BEAST (Drummond AJ and Rambaut A "BEAST: Bayesian evolutionary analysis by sampling trees" BMC EVOLUTIONARY BIOLOGY VOL 7, page 214).

A characteristic hallmark of sequence data for the influenza virus is the sampling density, with hundreds of isolates from the major circulating subtypes being sequenced every year. This is reflected in the low genetic and accordingly, evolutionary divergence of the corresponding sequence isolates.

**SUMMARY OF THE INVENTION**

It is the object of the present invention to provide phylogenies and genetic markers of viral fitness for genetic sequence data and their use for the selection of vaccine strains for seasonal influenza.

This object is solved by the subject matter of the independent claims.

Preferred embodiments are defined by the dependent claims.

In an embodiment, a method for identifying a phylogenetic tree for a plurality of biological sequences is provided. Each biological sequence is associated with a time stamp. First, distances between biological sequences of the plurality of biological sequences are obtained. Then, a weighted directed graph representation of the plurality of biological sequences is generated based on the obtained distances and the sampling dates associated with the plurality of biological sequences. Finally, a minimum spanning tree, MST, of the generated graph is computed and identified as the phylogenetic tree.

In another embodiment, a system for identifying a phylogenetic tree for a plurality of biological sequences, wherein each biological sequence is associated with a time stamp, includes a data preparer and a phylogeny identifier. The data preparer is communicatively coupled to the phylogeny identifier. The data preparer is adapted to obtain and output distances between biological sequences of the plurality of biological sequences. The phylogeny identifier comprises a graph generator and an minimum spanning tree, MST, identifier. The graph generator is adapted to generate a graph representation of the plurality of biological sequences based on the output distances from the data preparer and the time stamps associated with the plurality of biological sequences. The minimum spanning tree identifier is further adapted to compute an minimum spanning tree of the generated graph and identify the computed minimum spanning tree as the phylogenetic tree.

In a further embodiment, the computed phylogenetic tree forms the basis for the preparation of a vaccine, in particular to identify the expected viral strain for a forthcoming virus outbreak, which in turn forms the basis for the vaccine preparation.

In another embodiment, the preparation of a vaccine against a fast-evolving entity is based on a phylogenetic tree and includes the selection of an individual sequence and associated isolate from which the sequence was obtained for the entity; and the preparation of a vaccine based on the selected individual isolate.

Another embodiment pertains to the vaccine produced by the foregoing method.

In a further embodiment, the selection of an individual isolate of the fast-evolving entity comprises the steps of (i) computation of a phylogenetic tree from sequences available up to the point in time where the decision is to be made, (ii) identifying the number of repeated amino acid mutations occurring in each isolate and (iii) the selection of an isolate as a vaccine candidate when (a) the isolate sequence bears the maximum number of repeated amino acid mutations that occurred within a time-span of 4 years before the season in which the vaccine is to be produced, or (b) the isolate sequence bears the maximum number of repeated amino acid mutations that are located in antigenic sites and occurred within a time-span of 4 years before the season in which the vaccine is to be produced, or (c) the isolate sequence bears the maximum number of repeated amino acid mutations that cluster in the phylogeny, are located in antigenic sites and occurred within a time-span of 3 years before the season in which the vaccine is to be produced, or (d) the isolate sequence bears the maximum number of repeated amino acid mutations that cluster in the phylogeny, are located in sites determined experimentally to change the viral phenotype and occurred within a time-span of 4 years before the season in which the vaccine is to be produced.

**BRIEF DESCRIPTION OF THE DRAWINGS**

The accompanying drawings are incorporated into and form a part of the specification for the purpose of explaining the principles of the invention. The drawings are not to be construed as limiting the invention to only the illustrated and described examples of how the invention can be made and used. Further features and advantages will become apparent from the following and more particular description of the invention, as illustrated in the accompanying drawings, wherein:

FIG. 1          illustrates an exemplary method for constructing a phylogenetic tree;

FIGs. 2a to 2c    illustrates an exemplary method for generating a graph representation for a set of biological sequences;

FIG. 3          illustrates an exemplary system adapted to construct a phylogenetic tree;

FIGs. 4a to 4c    shows an exemplary phylogenetic tree constructed in accordance with an embodiment of the invention;

FIG. 5          shows the timeline and relative frequency for antigenic clades between 1993 and 2007 inferred from the phylogeny based on signature mutations of antigenic types;

FIG. 6          shows two types of mutation patterns which indicate unsampled evolutionary intermediate sequences.

**DETAILED DESCRIPTION OF THE INVENTION**

The illustrative embodiments of the present invention will be described with reference to the figure drawings, wherein like elements and structures are indicated by like reference numbers.

The present invention provides a new method and system for building phylogenetic trees (phylogenies) from biological sequences. For densely sampled sequence sets from a single evolving population such as the H3N2 subtype of seasonal influenza, a phylogeny which is identical or a close approximation in cost to a conventional maximum parsimony solution may be identified in at most quadratic time, which makes it applicable to data sets two to three orders of magnitude larger than what can be processed with conventional maximum parsimony techniques. The inferred trees may furthermore be multifurcating instead of bifurcating which is a more realistic representation of evolutionary relationships. Embodiments of the present invention are first to guarantee finding such a phylogeny, which is advantageous in particular for the study of fast evolving entities such as viruses from large sets of sequences.

A characteristic hallmark of sequence data for the influenza virus is the sampling density, with hundreds of isolates from the major circulating subtypes being sequenced every year. This is reflected in the low genetic and accordingly, evolutionary divergence of the corresponding sequence isolates.

The phylogenetic method of the present invention is suited for densely sampled sequences with low genetic divergence. One of the key differences between the present invention and common techniques is that instead of searching the tree space by tree-rearrangement operations for an optimal solution, ancestral intermediates are iteratively inferred based on characteristic mutation patterns in the phylogeny. For sequence sets of low genetic divergence such as those sampled from a single species, viral subtype or replicating cell line, this procedure allows to identify a phylogeny which is identical or a close approximation in cost to a maximum parsimony solution. The computing time rises at most quadratically with the number of input sequences, which makes the invention applicable to sets of sequences several orders of magnitude larger than what can be processed with conventional parsimony techniques. A further distinction is that the present invention allows to infer multifurcating tree topologies, which are a more realistic representation of the evolutionary relationships than bifurcating topologies returned by conventional approaches.

By applying methods and systems of the present invention to sequence data of seasonal influenza, subtype H3N2, repeatedly occurring amino acid changes in the inferred phylogeny are found that are significantly associated with the process of antigenic drift. These changes allow recommendation of suitable vaccine strains in seasons where an update of the vaccine components is required.

In short, an embodiment of the present invention provides a computer-implemented method and a computer system for identifying a phylogenetic tree from a plurality of biological sequences. Each biological sequence is associated with a sampling date. First, the plurality of biological sequences is aligned and a distance matrix is obtained. A subset of these sequences without any duplicated sequences is selected and a directed graph representation of the subset of biological sequences is generated based on the associated sampling dates. In some embodiments, the directed graph representation may be generated for the entire plurality of biological sequences. Then, a minimum spanning tree is computed from the weighted directed graph representation. In some embodiments, the sequences of unsampled evolutionary intermediates may be inferred in an update step in an iterative procedure from mutation patterns that reflect the difference in sequence between the nodes in the minimum spanning tree. The new sequences are added with associated time stamps to the sequence set. Then, sets of identical sequences may again be removed and an optimum branching (minimum spanning tree) may be recomputed. This step may be repeated until no new intermediates are found. In a final step, the sequences that have been set aside, if any, may be added to the plurality of sequences derived in the update step. From this plurality of sequences an optimum branching is computed and identified as the phylogenetic tree. Amino acid changes repeatedly occurring on the internal branches of the obtained tree can be used to identify sequences and associated viral isolates suitable as vaccine strains for the following influenza season.

An "isolate" is a sample of an entity that was isolated from a single source. The isolate represents a clonal subset of the entity, i.e. the isolate's sequence is derived from genetically identical individuals of the entity and the sequence of the isolate is unambiguous.

An "entity" is any organism, virus or replicating cell line. The entity can be a pathogen and either a microorganism or a virus. Microorganisms include bacteria, fungi, archaea, and protists, as well as microscopic plants such as plankton and animals such as amoeba. In an embodiment, the entity is a replicating cell line, such as a cancer cell line. In an embodiment, the entity is a virus. The virus may belong to the family of any one of Adenoviridae, Picornaviridae, Herpesviridae, Hepadnaviridae, Flaviviridae, Retroviridae, Orthomyxoviridae, Paramyxoviridae, Papovaviridae, Rhabdoviridae, or Togaviridae. Further, the entity may be selected from Influenzavirus A, Influenzavirus B, Influenzavirus C, Thogotovirus and Isavirus (all Orthomyxoviridae). In preferred embodiments the entity may subtypes of influenza virus A, such as H1N1, H1N2, H2N2, H3N1, H3N2, H3N8, H5N1, H5N2, H5N3, H5N8, H5N9, H7N1, H7N2, H7N3, H7N4, H7N7, H9N2 or H10N7. A "fast-evolving entity" is an entity that is capable of reproduce itself or, in the case of a virus, being reproduced within a short period of time. A short period of time is, for example, less than one week or less than one day.

The phylogenetic tree of the present invention can be multifurcating with nodes that have an arbitrary number of direct descendants. Thus, phylogenies for data with multiple or just one direct descendant(s) to any ancestral sequence can be identified.

The present invention also allows an intuitive visualization of evolutionary relationships along with epidemiological and experimental information and their study on the phylogenetic tree. The method and system of the present invention were evaluated with a study of the major surface protein of the influenza A virus (subtype H3N2) over the time span from 1993 to 2007. H3N2 is the most rapidly evolving variant of endemic influenza and was introduced in the human population in 1968 with the Hong Kong pandemic. Phylogenetically, influenza A belongs to the family of *Orthomyxoviridae* and features a single-stranded RNA genome composed of eight distinct segments totaling approximately 11 kb in length.

Through continuous change (antigenic drift) in the epitope sites of the major surface proteins, hemagglutinin (HA) and neuraminidase (NA), the virus is able to evade immune recognition in previously primed individuals. Thus, one may incur multiple infections over the course of several years, despite existing immunity to older variants, and repeated updates of vaccines with novel strains are required. Due to the complex interplay of factors shaping antigenic drift and viral fitness (high mutation rates, global seasonal spread and interaction with the inherently variable acquired immune response), understanding the process of antigenic drift is still a key target in influenza research today.

A phylogenetic tree or phylogeny, sometimes also called an evolutionary tree, is a tree showing the evolutionary relationships among various biological isolates that are believed to have a common ancestor. A rooted phylogenetic tree is a directed tree with a unique node corresponding to the most recent common ancestor of all the biological isolate sequences at the leaves or internal nodes of the tree. Unrooted trees, on the other hand, illustrate the relatedness of the nodes without making assumptions about common ancestry.

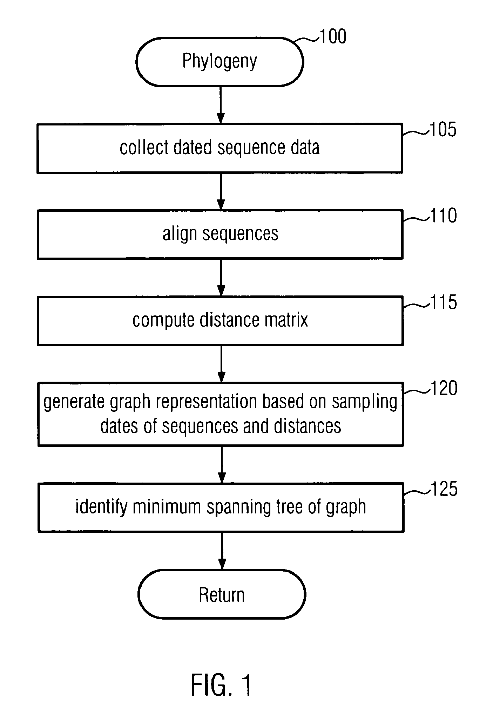

Referring to FIG. 1, unlike conventional techniques for identifying phylogenetic trees, embodiments of the present invention may provide a multifurcating instead of a bifurcating phylogenetic tree and is applicable to sequence sets two to three orders of magnitude larger than what can be processed with conventional maximum parsimony techniques.

Embodiments of the present invention may be applied to identify a phylogenetic tree for any set of biological sequences of interest. A biological sequence (also referred to as sequence) is the primary structure of a biopolymer. For instance, a biological sequence may be the order of the nucleotide bases in a DNA or RNA molecule or the order of amino acids in a protein. The sequences that are subject to the phylogenetic analysis may be ribonucleic acid sequences (RNA sequences), deoxyribonucleic acid sequences (DNA sequences) or amino acid sequences (protein sequences).

Accordingly, at step 105, data regarding biological sequences of the biological entity of interest, such as a virus or any other entity, is collected. In an embodiment, dated sequence data is collected, i.e. each biological sequence is associated with a sampling date or a time stamp. The sequences can be derived from public databases or from any other source. This may be done by accessing a central database such as the Influenza Sequence Database at NCBI (National Center for Biotechnology Information) or a database of the WHO (World Health Organization), where biological sequence data is stored.

In an embodiment, the biological sequences are nucleic acid sequences encoding a protein, or protein sequences of the entity. The protein may be at least partially exposed to the surface of the fast-evolving entity and may contain at least one antigenic site. An antigenic site is an epitope of a protein, said epitope being able to elicit an immune response of the host to said epitope. The epitope may be surface-exposed. In a preferred embodiment, the virus may be an Influenza virus and the sequences used for the determination of the phylogeny may be selected from hemagglutinin (HA) or neuraminidase (NA).

In an embodiment, the sampling date/time stamp associated with a biological sequence may correspond to the date on which the biological sequence has been determined from the isolate of the biological entity. Alternatively, the sampling date/time stamp may correspond to the date on which the (isolate of the) biological entity was sampled. If the database provides a sampling date/time stamp for a biological sequence, this date may be collected together with the biological sequence data. If the actual sampling date of a biological sequence is not available, a sampling date may be estimated.

In other embodiments, only partial information on sampling dates for biological sequences may be available. For instance, the sampling date may be specified with a resolution of a certain time span, such as months or years. Hence, in these embodiments, the sampling date/time stamp associated with a biological sequence may correspond to a time span. As an example, but not a limitation, for each isolate the day, month and year of the isolate and its corresponding sequence may be known. It is, however, sufficient that the year or both, the season (e.g. winter season) and the year, is specified for each isolate. In those instances where only the year of the isolate is known, the date may be set to the season in which the entity is predominantly active, e.g. if it is an influenza A isolate from the northern hemisphere, the date is set to the winter season of the year of the isolate. The winter season is defined as ranging from 1st October to 31st March for the northern hemisphere and from 1st April to 30th September for the southern hemisphere.

Furthermore, each isolate may be attributed with its geographic origin (e.g. the place of the isolation of the sequence, or preferably the place where the individual from which the sequence was isolated was infected with the fast-evolving entity). The isolates can be classified as belonging to the northern hemisphere, the southern hemisphere or as being of tropical origin. The tropical area is defined as being centered on the equator and limited in latitude in the northern hemisphere, at 23°26' (23.4°) N latitude, and in the southern hemisphere at 23°26' (23.4°) S latitude. The geographic regions outside the tropics belong to the northern and southern hemisphere. However, any other suitable geographic classification (e.g. by country or continent) may be used for the attribution of localization data.

At 110, the biological sequences derived from the data collected at 105 may be aligned. In bioinformatics, sequence alignment is a way of arranging the primary sequences of DNA, RNA, or protein to identify regions of identity and/or similarity. The alignment may be performed with any common alignment technique. In an embodiment, pairwise alignment techniques are used to find the best-matching piecewise (local) or global alignments of two biological sequences. As an example, but not a limitation, conventional pairwise alignment techniques such as dot-matrix techniques, dynamic programming and word techniques may be used. According to alternative embodiments, multiple sequence alignment techniques may be used. A multiple sequence alignment is a sequence alignment of three or more biological sequences, generally protein, DNA, or RNA. Multiple alignment techniques align all of the biological sequences under consideration. In some embodiments, both pairwise and multiple sequence alignment techniques may be used. Multiple alignment software is known in the art. For example, ClustalW (Higgins, D.G., Bleasby, A.J. and Fuchs, R. 1992 CLUSTAL V: improved software for multiple sequence alignment. Computer Applications in the Biosciences, volume 8, number 2, pages 189-191) can be used to generate a multiple alignment. The underlying algorithms for establishing multiple sequence alignments are known in the art and can be based on e.g. dynamic programming techniques, clustering methods, Hidden Markov model-based techniques and methods that use sequence patterns or conserved motifs to anchor parts of the multiple alignment. By way of example and not a limitation, ClustalW was used. However, any program or algorithm can be used as long as it is capable of establishing a multiple sequence alignment from biological sequences.

In typical usage, protein alignments use a substitution matrix to assign scores to amino-acid matches or mismatches, and a gap penalty for matching an amino acid in one sequence to a gap in the other. DNA and RNA alignments may use a scoring matrix, but in practice often simply assign a positive match score, a negative mismatch score, and a negative gap penalty.

After having aligned the sequences under consideration, such as the sequences collected at step 105, a distance matrix for the biological sequences may be computed at 115. Distance matrices rely on a measure of the distance between biological sequences. Distance may be defined as the fraction of mismatches at aligned positions, with gaps being ignored, counted as mismatches or otherwise weighted. In embodiments of the invention, the distance matrix may be computed using exact matching without distance correction and no weights for gaps. However, any other technique for computing a distance matrix may be used.

In more detail, a distance matrix is a matrix (two-dimensional array) containing the distances, taken pairwise, of a set of points. In this case a distance matrix can be a similarity matrix. Nucleotide similarity matrices are used to align nucleic acid sequences. For example, a simple matrix will assign identical bases a score of +1 and non-identical bases a score of -1. A more complicated matrix may give a higher score to transitions (changes from a pyrimidine such as C or T to another pyrimidine, or from a purine such as A or G to another purine) than to transversions (from a pyrimidine to a purine or vice versa). The match/mismatch ratio of the matrix sets the target evolutionary distance (States et al. 1991 METHODS - A companion to Methods in Enzymology 3:66-70); the +1/-3 DNA matrix used by BLASTN is best suited for finding matches between sequences that are 99% identical; a +1/-1 (or +4/-4) matrix is much more sensitive as it is optimal for matches between sequences that are about 70% identical. For protein sequences a PAM series of matrices may be used. PAM matrices are labeled based on how many nucleotide changes have occurred, per 100 amino acids. PAM matrices are most useful at short evolutionary distances (PAM10 - PAM120). At long evolutionary distances, for example PAM250 or at only 20% identity, BLOSUM matrices are more effective.

In some embodiments, a computed distance matrix for a set of biological sequences may be obtained from a database instead of actually computing the distance matrix from raw sequence data. In those embodiments, steps 105, 110 and 115 may be omitted. The distance matrices stored in the database may have been computed in accordance with the computation of step 115. In still other embodiments, only distances needed for the subsequent steps of method 100 may be obtained from a database. In those embodiments, steps 105 (at least partially), 110 and 115 may also be omitted.

In other embodiments, data regarding an aligned set of biological sequences may be obtained from a database, such that steps 105 (at least partially) and 110 may be omitted. The alignment of the sequences stored in the database may have been done in accordance with step 110.

In embodiments of the invention, the distance matrix may be obtained from a number of different sources, including measured distance (for example from immunological studies) or morphometric analysis, various pairwise distance formulae (such as Euclidean distance) applied to discrete morphological characters, or genetic distance from sequence, restriction fragment, or allozyme data. Further, raw distance values may be calculated by simply counting the number of pairwise differences in character states of the sequences (Manhattan distance).

At step 120, a graph representation of the biological sequence data is generated based on the sampling dates/time stamps associated with the biological sequences and distances between the biological sequences. In embodiments of the invention, the distances may be derived from a distance matrix, which was either computed in step 115 or otherwise obtained.

In some embodiments, step 120 may have an initializing step, where for each set of identical sequences, all but one instance associated with the earliest sampling date/time stamp may be set aside before generating the graph representation. The retained sequence may also be the sequence associated with the latest sampling date/time stamp or a sequence chosen by any other criterion. This results in a sequence set without any duplicated sequences. Thus, in the initializing step, identical biological sequences in the set of biological sequences of interest may be determined. Then for each set of identical sequences, the biological sequence from the set of identical sequences associated with the earliest (or latest or selected according to any other criterion) sampling date/time stamp may be selected for further processing at step 120. In addition, all unique biological sequences may be selected for step 120.

In the context of the present invention, the graph representation may be a directed graph. A directed graph is a set of nodes connected by directed edges. Thus, a graph $G$ is an ordered pair $G := (V,E)$, where $V$ is a set whose elements are called nodes and $E$ is a set of ordered pairs of nodes called directed edges.

In the present invention, let $V = \{v_1, \ldots, v_n\}$ be the set of nodes representing the input sequences, e.g. the sequences collected at step 105. Each node $v_i$ represents a biological sequence which is associated with a specific sampling date/time stamp $t_i$ such that $T = \{t_1, \ldots, t_n\}$ is the set of sampling dates/time stamps. In some embodiments, the sequence associated with the earliest sampling date/time stamp may be identified and the node representing this sequence may be selected as root node. Then, for each node, outgoing edges are added to all nodes with a later sampling date/time stamp resulting in the set of edges $E = \{(v_i, v_j) : t_i < t_j\}$. Accordingly, an outgoing edge from a first node to a second node may be added, if the sampling date/time stamp associated with the biological sequence represented by the second node is later than the sampling date/time stamp associated with the biological sequence represented by the first node.

In the context of the present invention, an outgoing edge of a node $v_i$ is a directed edge connecting the node $v_i$ with another node and being directed from the node $v_i$ to the other node. Similarly, an incoming edge of a node $v_i$ is a directed edge connecting the node $v_i$ with another node and being directed from the other node to the node $v_i$.

The Directed Acyclic Graph (DAG) generated in step 120 has at least ($n$-1) edges, if all sequences except the sequence being represented by the root node are associated with the same sampling date/time stamp. If every sequence is associated with a different sampling date/time stamp, the DAG generated during step 120 has $\dfrac{n \cdot (n-1)}{2}$ edges.

A DAG is a directed graph with no directed cycles, i.e. for any node $v_i$, there is no non-empty directed path that starts and ends on $v_i$. A path in a graph is a sequence of nodes such that from each node there is an edge to the next node in the sequence. The first node is called the start node and the last node is called the end node. Hence, a cycle is a path such that the start node and end node are the same.

To generate a weighted DAG, the edges of the graph computed in 120 are weighted. In embodiments of the invention, the edge weight of an edge may correspond to the distance between the biological sequences represented by the nodes connected by this edge. The distance between the biological sequences may be derived from the obtained/ computed distance matrix or from any other source providing a distance for the biological sequences. In some embodiments, the distance between two biological sequences may be retrieved, e.g. from a public database or any other source, when an edge between nodes representing these two biological sequences is added or weighted.

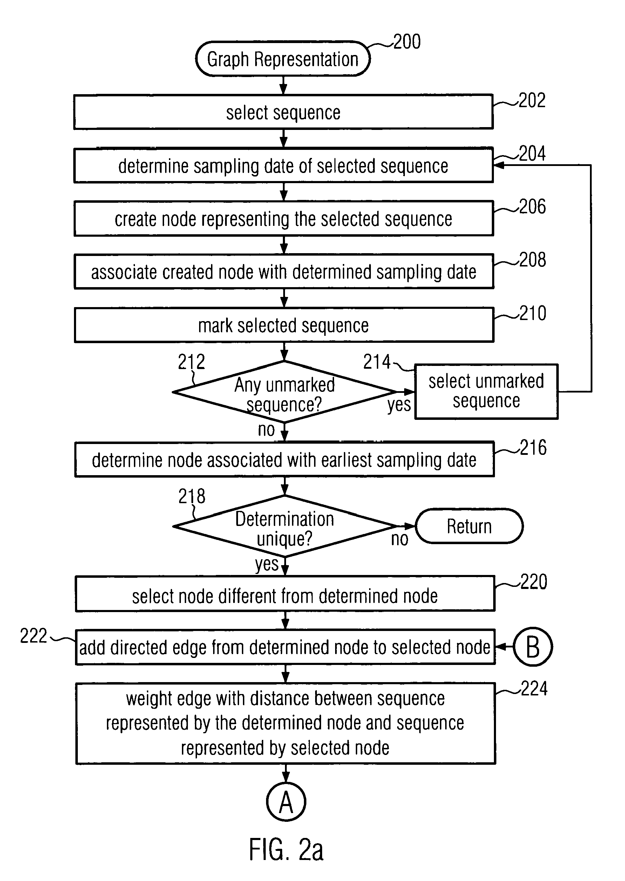

The details of the computation of the weighted DAG representing the biological sequences under consideration (step 120 of FIG. 1) will be described in more detail with respect to FIGs. 2a to 2c.

At 125, a minimum spanning tree (MST), also referred to as optimum branching, in the graph computed at step 120 is identified. A tree is a graph in which any two nodes are connected by exactly one path. Thus, a tree with n nodes has ($n$-1) edges. A spanning tree of a connected graph is a subgraph which is a tree and connects all the nodes of the graph. A single graph may have many different spanning trees. If a weight is assigned to each edge of the graph, these weights may be used to assign a weight to a spanning tree. As an example, but not a limitation, the weight of a spanning tree may be the sum of the weights of all the edges forming the spanning tree. An optimum branching or minimum spanning tree or minimum weight spanning tree of a directed graph may be a spanning tree with weight less than (or equal) to the weight of any other spanning tree in this graph.

There are several algorithms for identification of a minimum spanning tree in a weighted directed graph in quadratic time such as the Chu-Liu/Edmonds algorithm. However, the minimum spanning tree identified in step 125 may be computed with any available algorithm including, but not limited to the Chu-Liu/Edmonds algorithm or Bock's algorithm

According to an embodiment of the invention, a minimum spanning tree of the graph generated at step 120 is constructed by selecting for each node in the graph the incoming edge with the lowest assigned weight (distance). In case of multiple incoming edges having assigned the lowest weight, the incoming edge starting from the node representing the biological sequence associated with the latest sampling date/time stamp may be chosen from the group of edges having the lowest weight. Alternatively, in case of multiple incoming edges having been assigned the lowest weight, the incoming edge whose source node represents the biological sequence associated with the earliest sampling date/time stamp may be selected from the group of edges having the lowest weight. In still other embodiments, in case of multiple incoming edges having been assigned the lowest weight, the incoming edge may be selected randomly from the group of edges having the lowest weight. Unselected edges may be removed from the graph.

As the graph generated at step 120 is acyclic, no cycles need to be resolved.

In some embodiments, the resulting minimum spanning tree may be selected as the phylogenetic tree for the set of biological sequences under consideration. Thus, a phylogenetic tree constructed with the method 100 is the phylogenetic tree for the biological sequences under consideration with the least overall weight (cost) with respect to the defined distances between the biological sequences among all possible phylogenetic trees for those biological sequences. In contrast to conventional techniques for constructing phylogenetic trees, the method 100 results in phylogenetic trees that may be multifurcating instead of bifurcating.

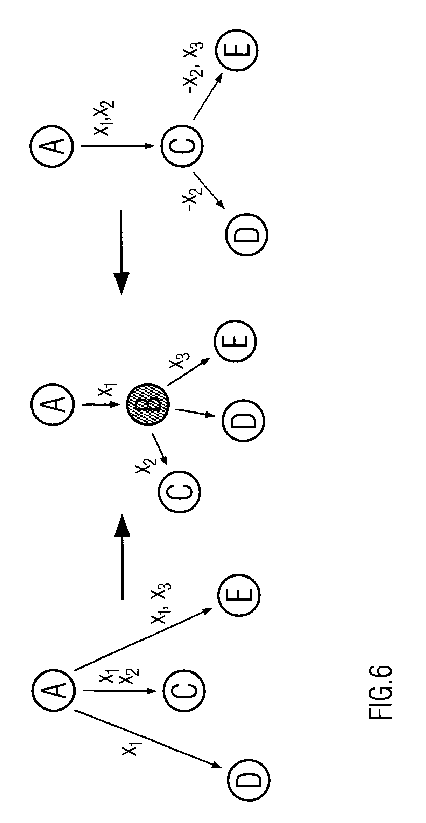

In some embodiments, the resulting minimum spanning tree from method 100 may be an initial optimum branching. In an update step, sequences of unsampled evolutionary intermediates are inferred based on differences between sequences represented by nodes connected by an edge of the initial minimum spanning tree. Then, time stamps are assigned to the inferred unsampled evolutionary intermediates (the details on inferring unsampled evolutionary intermediates and assigning time stamps to the inferred intermediates will be discussed below with respect to FIG. 6). The inferred sequences are added with assigned time stamps to the sequence set under consideration and steps 120 and 125 as described above may be performed for this set of sequences. This update step may be repeated until no new intermediates are found (inferred).

For embodiments where step 120 comprises the initializing step, the sequences not selected at each of steps 120 (the update step is an iterative process repeating steps 120 and 125) are added to the sequence set derived in the update step, if no new evolutionary intermediates are found. From this plurality of sequences an optimum branching may be computed according to steps 120 and 125 and the resulting minimum spanning tree may be identified as the phylogenetic tree.

According to embodiments of the invention, the method 100 may also be used if only partial information on sampling dates is available, i.e. if the sampling date is only specified with a resolution of a certain time span such as months or years. In this case, all nodes representing biological sequences associated with sampling dates/time stamps within the same time span may be connected in the graph generated during step 120. However, the graph resulting from this computation is not an acyclic graph. Therefore, cycles in the graph are resolved by computation of an minimum spanning tree from weighted directed graphs as described in R.E. Tarjan, Networks, volume 7, pages 25-35. This may be done at step 125 during the computation of the minimum spanning tree.

According to embodiments of the invention, the method 100 may also be used if no information on the sampling dates is available. In this case, all nodes representing biological sequences are connected with undirected edges to each other and a least cost tree or minimum spanning tree may be computed from the resulting weighted undirected graph using a minimum spanning tree algorithm for undirected graphs such as Prim's algorithm, Kruskal's algorithm, Borůvka's algorithm, the reverse-delete algorithm or the algorithm developed by Bernard Chazelle. This may be done at step 125 during the computation of the minimum spanning tree.

Referring now to FIGs. 2a to 2c, details of the graph generation step 120 of method 100 according to some embodiments of the invention are described. At 202, data representing a biological sequence is selected. In some embodiments, this selection may be done randomly, while in other embodiments, the biological sequence associated with the earliest/latest sampling date/time stamp may be selected. If the sequence was selected randomly, the associated sampling date/time stamp is determined at step 204. In embodiments where the sequence is selected according to its sampling date/time stamp, the sampling date/time stamp may already be determined at step 202.

At 206 a node is created representing the selected biological sequence. This node is associated with the determined sampling date/time stamp of the selected sequence at 208. The so processed biological sequence data may be marked as being represented by a node at 210.

At 212 it is determined whether any unmarked biological sequence data remains in the set of biological sequences under consideration. If there is unmarked biological sequence data, unmarked biological sequence data representing a biological sequence not yet represented by a node is selected at 214 and the method branches back to step 204.

However, if it is determined at 212 that there is no unmarked biological sequence, i.e. all biological sequences in the set of biological sequences of interest are represented by a node, the method 200 branches to step 216, where the node representing the biological sequence data associated with the earliest sampling date/time stamp is determined.

At 218, it is determined whether the determination performed in step 216 results in a unique node. If the determination results are not unique, the method may abort and return an error. In alternative embodiments, a node may be randomly selected from the group of nodes representing biological sequences sharing the earliest sampling date/time stamp while the other nodes in the group of nodes representing biological sequences sharing the earliest sampling date/time stamp may be left unconsidered in the following steps 220 to 256. In some embodiments, each node from the group of nodes representing biological sequences sharing the earliest sampling date/time stamp may be selected and for each selected node, the following method steps 220 to 256 may be performed, wherein the respective unselected nodes in the group of nodes representing biological sequences sharing the earliest sampling date/time stamp may be left unconsidered. In those embodiments, as many graph representations as nodes in the group of nodes representing biological sequences sharing the earliest sampling date/time stamp may be generated.

If it is determined at 218 that the determination of 216 results in a unique node, a node different from the node determined at 216 is selected at 220. In cases, where the results of the determination at 216 were not unique and a node was selected randomly at 218, this randomly selected node corresponds to the determined node.

At 222 a directed edge (outgoing edge with the determined node as source node and the first node as sink node) is added from the determined node to the selected node. At 224 the distance between the biological sequences represented by the determined node and the selected node is assigned to the added directed edge as edge weight. The distance between the biological sequences may be derived from the obtained/computed distance matrix (as, for instance, described with respect to step 115 of FIG. 1) or from any other source providing a distance for these biological sequences.

At 226 the selected node may be marked as considered with respect to the determined node. At 228 it is determined whether there is any node that is not considered with respect to the determined node, i.e. not marked as considered with respect to the determined node. If it is determined at 228 that there are unconsidered nodes, the method proceeds to 230, where a further node different from the node determined at 216 is selected from the group of nodes that are not marked as considered. Then the method branches back to step 222.

If it is, however, decided at 228 that all nodes have been considered with respect to the node determined at 216, the method proceeds to 232 and marks the determined node as done.

At 234 a node from the group of undone nodes is selected as first node. This selection may be random or under consideration of the sampling date/time stamp associated with sequences represented by the nodes, i.e. the earliest sampling date/time stamp associated with the biological sequences represented by nodes in the group of undone nodes may be identified and a node representing a biological sequence associated with this earliest sampling date/time stamp may be selected as first node. If the first node was randomly selected at 234, the sampling date/time stamp associated with the biological sequence represented by that node may be determined at 236. In embodiments where the first node is selected under consideration of the sampling date/time stamp, the sampling date/time stamp of the biological sequence represented by the first node may be determined at step 234.

At 238 a node different from the first node and different from the node determined at 216 is selected as second node. In embodiments, where the first node was selected under consideration of the sampling date/time stamp, i.e. the earliest sampling date/time stamp associated with the biological sequences represented by nodes in the group of undone nodes was identified and a node representing a biological sequence associated with this earliest sampling date/time stamp was selected as first node at 234, the second node may be selected from the group of undone nodes, wherein the group of undone nodes does not include the first node such that the second node is different from the first node. At 240 the sampling date/time stamp associated with the biological sequence represented by the second node is determined and compared at 242 with the sampling date/time stamp associated with the biological sequence represented by the first node.

If the sampling date/time stamp associated with the biological sequence represented by the first node is not earlier than the sampling date/time stamp of the biological sequence represented by the second node, the method branches from 242 to step 248.

However, if the sampling date/time stamp associated with the biological sequence represented by the first node is earlier than the sampling date/time stamp of the biological sequence represented by the second node, the method branches from 242 to step 244, at which an outgoing edge is added from the first node to the second node. This newly added edge is weighted at 246 with the distance between the biological sequences represented by the first and second node. The distance between the biological sequences may be derived from the obtained/computed distance matrix (as, for instance, described with respect to step 115 of FIG. 1) or from any other source providing a distance for these biological sequences.

At 248 the second node is marked as considered with respect to the first node. At 250 it is decided whether there are any nodes not considered with respect to the first node, i.e. not marked as considered with respect to the first node.

If there are unconsidered nodes with respect to the first node, a node different from the node determined to represent the biological sequence being associated with the earliest sampling date/time stamp (step 216) and the first node is selected from the group of nodes not marked as considered with respect to the first node as the second node at 252. Then the method branches back to step 240.

In embodiments, where the first node was selected under consideration of the sampling date/time stamp at 234, i.e. the earliest sampling date/time stamp associated with the biological sequences represented by nodes in the group of undone nodes was identified and a node representing a biological sequence associated with this earliest sampling date/time stamp was selected as first node at 234, the second node may be selected from the group of undone, unconsidered nodes not including the first node such that the second node is different from the first node.

If all nodes have been considered with respect to the first node, the first node is marked as done at 254. At 256 it is decided whether all created nodes are done. If there is any undone node, the method branches back to 234. However, if all nodes are done, the method returns.

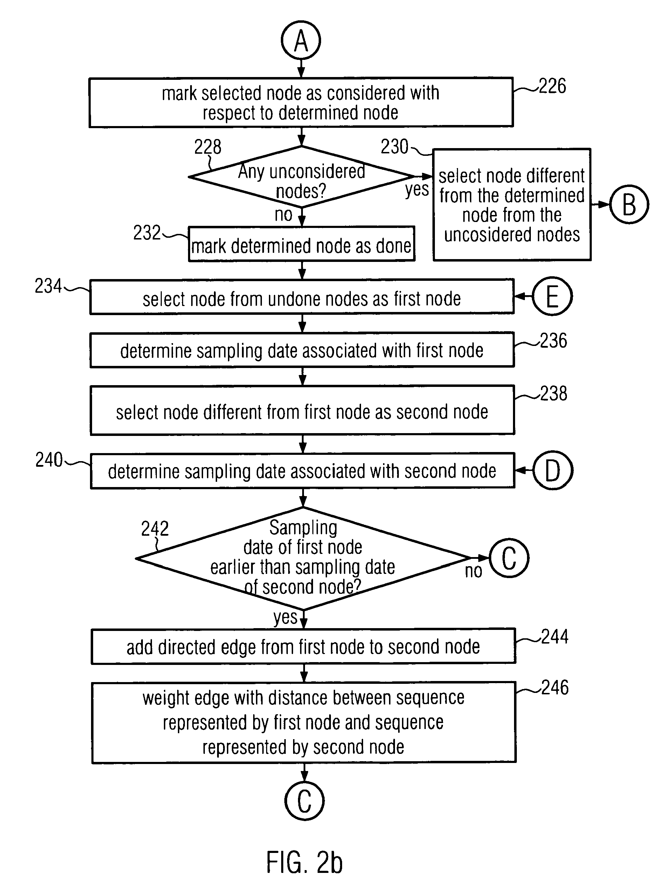

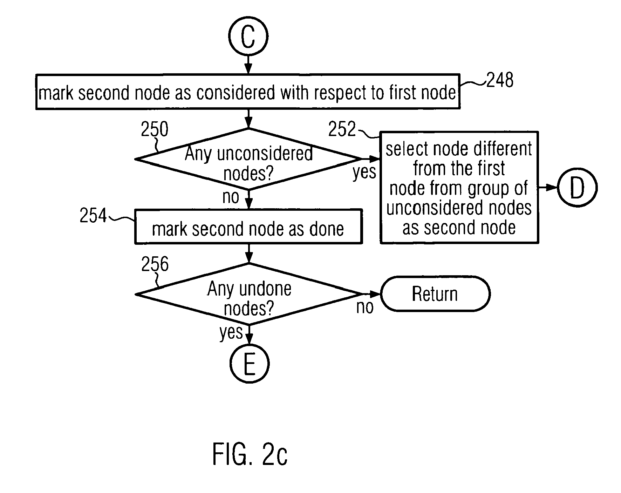

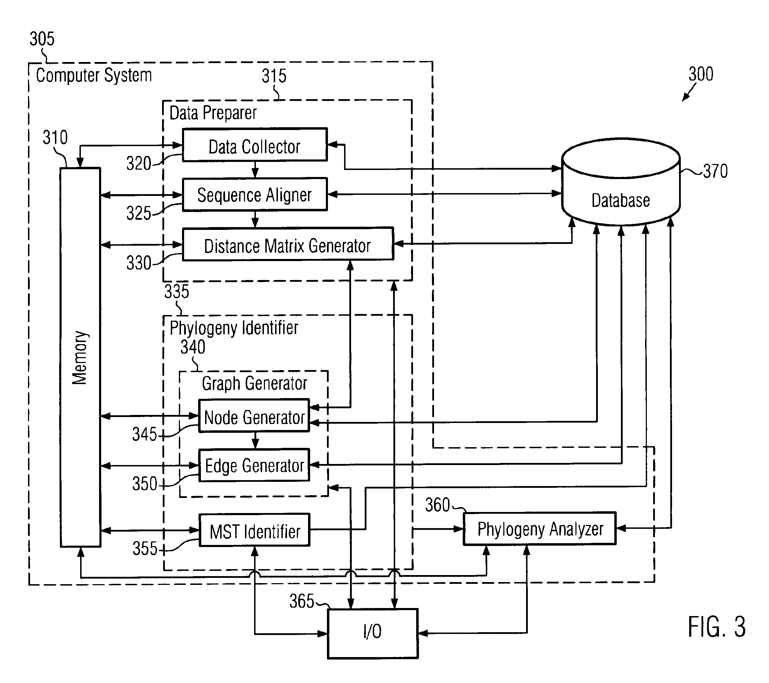

Turning now to FIG. 3, an exemplary system 305 adapted to carry out the methods described with respect to FIGs. 1 and 2 is described. The system 305 may be a general purpose computer system such as a mainframe computer, a workstation, a personal computer, a laptop or any other suitable computing device. In an embodiment of the present invention the computer system 305 may comprise a memory 310, a data preparer 315, a phylogeny identifier 335 and optionally, a phylogeny analyzer 360. The memory 310, the data preparer 315, the phylogeny identifier 335 and the phylogeny analyzer 360 may be communicatively coupled to each other, for instance via an internal bus system, for data exchange. In addition, the data preparer 315, the phylogeny identifier 335 and the phylogeny analyzer 360 may output data via an interface to external output devices 365 such as a display or a plotter. The modules 315, 335 and 360 may also receive input via an interface from external input devices 365 such as a keyboard or a mouse. In some embodiments, the modules 315, 335 and 360 may additionally or alternatively store their outputs to or receive their inputs from a computer-readable medium, accessible via an input/output device 365. In some embodiments, the memory 310 may also be couple to input/output (I/O) devices 365 to communicate with those device. In those embodiments, the modules 315, 335 and 360 may also output data to and receive instructions from the I/O devices 365 via the memory 310.

In addition, the computer system 315 may be coupled to an external database 370, for instance, via the internet or any other network. In those embodiments, each of the modules 315, 335 and 360 may retrieve input data from the database and/or transmit results to the database 370. Alternatively or in addition, the modules 315, 335 and 360 may communicate with the database 370 via memory 310 which is also coupled to the database 370 in those embodiments.

The database 370 may store results from modules 315, 335 and 360 for later retrieval for computer systems similar to system 305 that are coupled to the database 370. Additionally, the database may store data needed for the computations carried out on computer system 305, such as biological sequences, sampling dates or time stamps associated with the biological sequences, sets of aligned biological sequences, distances between biological sequences, distance matrices for sets of biological sequences, graphs for a set of biological sequences generated in accordance with step 120 of FIG. 1 and/or method 200 of FIGs. 2a to 2c, and phylogenetic trees built in accordance with method 100.

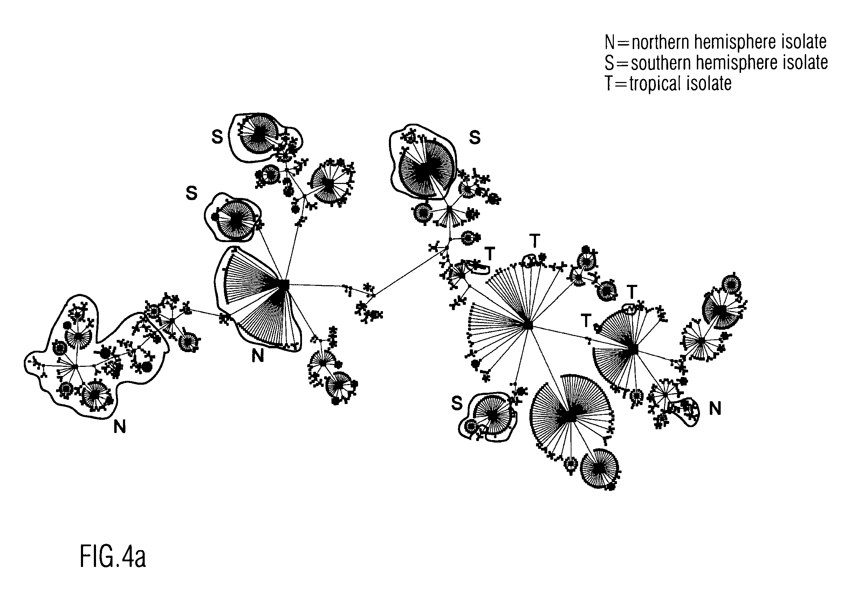

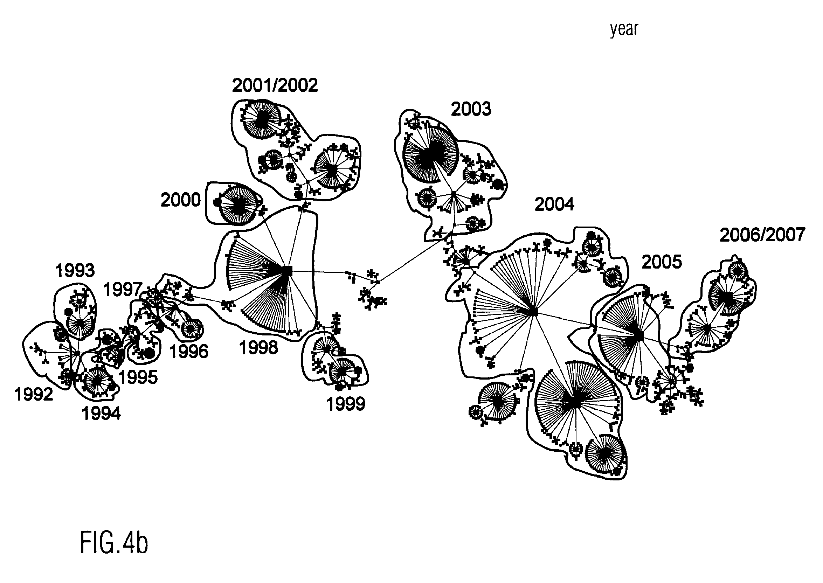

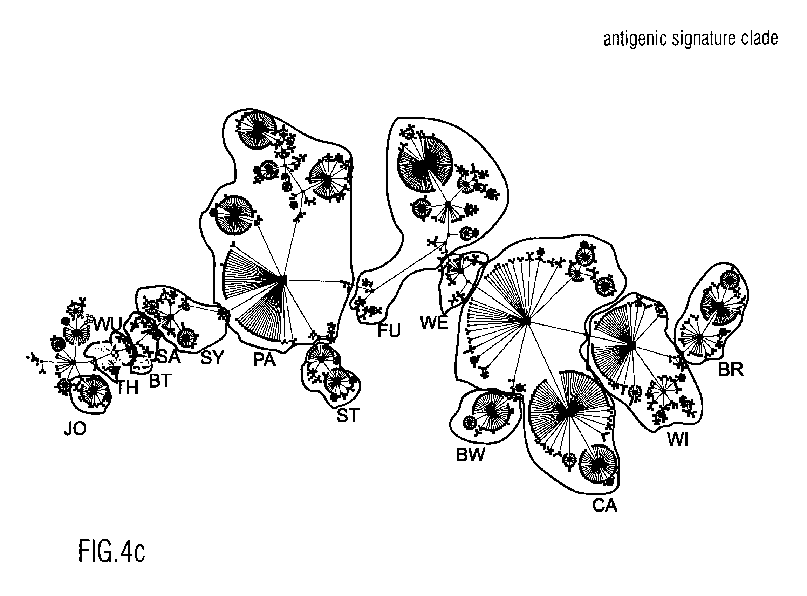

In embodiments of the invention, the data preparer 315 may be adapted to carry out steps 105 to 115 of FIG. 1 and the phylogeny identifier 335 may be adapted to carry out steps 120 and 125 of FIG. 1 as well as method 200 of FIGs. 2a to 2c. In some embodiments, the computer system 305 may additionally comprise the phylogeny analyzer 360, which is adapted to interpret the built phylogenetic trees as described below with respect to FIGs. 4 to 5.

The data preparer 315 may comprise a data collector 320, a sequence aligner 325 and a distance matrix generator 330. The data collector 320 may be adapted to perform step 105 of method 100 and thus may be coupled to the database 370 to retrieve data needed by the sequence aligner 325, the distance matrix generator 330, the phylogeny identifier 335 and/or the phylogeny analyzer 360. The data collector 320 may directly provide modules 325, 330, 335 and/or 360 with the collected data or indirectly via the memory 310.

The sequence aligner 325 may be adapted to perform step 110 of method 100. To that end, it may retrieve biological sequence data directly from the external database 370 or from the data collector 320.

The distance matrix generator 330 may be adapted to perform step 115 of method 100. In some embodiments, module 330 may compute distances between aligned sequences provided by the sequence aligner 325 or by the external database 370 and based on these distances generate a distance matrix. In other embodiments, the distance matrix generator 330 may additionally or alternatively retrieve distances between biological sequences from external database 370. In still other embodiments, the data collector 320 may retrieve a distance matrix for a set of biological sequences from the database 370 or retrieve distances between biological sequences from external database 370 on a request from the phylogeny identifier 335 without actually providing a complete distance matrix. In those embodiments, the sequence aligner 325 and/or the distance matrix generator 330 may be inactive.

The data preparer 315 outputs a distance matrix or distances between biological sequences to the phylogeny identifier 335 and/or the memory 110. In some embodiments, the data preparer 315 may also transmit the computed distance matrix to the external database 370.

The phylogeny identifier 335 is adapted to retrieve data relating to distances between biological sequences and sampling dates or time stamps relating to the biological sequences from the data preparer 315, the memory 110 and/or directly from the external database 370. The phylogeny identifier 335 may comprise a graph generator 340 adapted to perform step 120 of method 100 and/or method 200, as well as an MST identifier 355 adapted to perform step 125 of method 100.

The graph generator 340 may comprise a node generator 345 adapted to perform steps 202 to 214 of method 200 and an edge generator 350 adapted to perform steps 216 to 256 of method 200. Both the node generator and the edge generator may be communicatively coupled to the memory 110 and/or the external database 370 to provide results to or retrieve data from module 110 or the database 370. In some embodiments, the graph generator 340 may be adapted to obtain graphs from the database 370. The graph generator may also be coupled to the I/O devices 365 to display or output the generated graph. Also, the graph generator 340 may retrieve data from or output data to a computer-readable medium accessible via an I/O device 365.

The minimum spanning tree identifier 355 may be coupled to the memory 310, the graph generator 340, the I/O devices 365 and/or the external database 370. The MST identifier 355 may be adapted to compute a minimum spanning tree in accordance with step 125 of method 100. To this end, the MST identifier 355 may retrieve a graph from the graph generator 340, the memory 310 or the database 370 and output the identified MST to the memory 110, the I/O devices 365, the external database 370 and/or the phylogeny analyzer 360.

The phylogeny analyzer 360 is communicatively coupled to the database 370, the phylogeny identifier 335, the memory 310 and the I/O devices 365. The phylogeny analyzer is adapted to interpret phylogenetic trees with a consistent timeline built by the phylogeny identifier 335 or retrieved from the database 370 as described in more detail below with respect to FIGs. 4 to 5.

The invention according to methods 100 and 200 may be described in the general context of computer-executable instructions, such as program modules, being executed by a computer such as computer 305. Hence the method 100 and 200 may be computer-implemented. Generally, program modules include routines, programs, objects, components, data structures, etc. that perform particular tasks or implement particular abstract data types. The general purpose or special purpose computing system environments or configurations may be programmable using a high-level computer programming language. In some embodiments, the general purpose or special purpose computing system environments or configurations may also use specially-programmed, special-purpose hardware.

The invention may also be practiced in distributed computing environments where tasks are performed by remote processing devices that are linked through a communication network. In a distributed computing environment, program modules may be located in both local and remote computer storage media including memory storage devices.

In the research of disease and drug resistance, sequences are increasingly becoming available for isolates that have been sampled at successive points in time. Embodiments of the present invention find phylogenies based on characteristic mutation patterns of unsampled evolutionary intermediates for this data. The evolution of the major surface protein and antigenic determinant of influenza A for the time period from 1993 to 2007 was studied with methods of the present invention, using all available dated sequences. The analysis of repeatedly occurring mutations in the phylogeny revealed a significant association with antigenic drift and their suitability as genetic markers of viral fitness and identification of vaccine strain candidates. The phylogeny is divided in distinct clades representing the major antigenic types of the virus for the analyzed time period. The present invention allows for studying evolution from large-scale data sets and returns multifurcating tree topologies which visualize the genetic diversity and geographic prevalence of emerging lineages..

Antigenic lineages: Between 1993 and 2007, several distinct antigenic types of H3N2 have emerged in the human population by antigenic drift of the epitope sites. The genetic changes of novel strains relative to previous reference strains are continuously monitored and reported by the influenza centers of the WHO. As antigenic types have only been reported for very few sequences, the signature mutations are used to partition the data and identify phenotype-related clades in the phylogeny. All analyzed mutation signatures (lists of mutations) for the different antigenic types of influenza subtype H3N2 during the analyzed time period (1993-2007) are given in the following table (Table 2).

Table 1: Mutation signatures described by the World Health Organization in their semiannual reports for antigenic types of influenza A, subtype H3N2 between 1993 and 2007. Positions refer to the HA1 subunit of the hemagglutinin protein of influenza A, subtype H3N2. Mutations describe the differences of a new predominant antigenic type relative to the previous predominant antigenic type.

| Type | Mutation signature |
|------|--------------------|
| Johannesburg (JO)$_e$ | S47P, D124N, S219Y |
| Thessaloniki (TH)$_d$ | G172D, F216N, F219S, S278N |
| Wuhan (WU) | K135T, N145K, N262S |
| Bratislava (BT)$_a$ | R57Q, K92T, N122K, G275D |
| South Africa (SA) | T121N/T, G124S, D133N, G142R/G |
| Sydney (SY) | K62E, K156Q, E158K, V196A, N276K |
| Panama (PA) | I144N/D, K172E, T192I |
| Stockholm (ST)$_{c, e}$ | G5V, Q33H, K92T, N144I, D271 N |
| Fujian (FU) | L25I, H75Q, H155T, Q156H/Q, A131T, V202I, G225D |
| Wellington (WE) | Y159F, S189N, S227P |
| California (CA) | Y159F, S189N, V226I, S227P |
| Baden-Wuerttemberg (BW)$_b$ | V112I, N145S, Y159F, K173E, S189N, V226I, S227P |
| Wisconsin (WI) | S193F, D225N |

(continued)

| Type | Mutation signature |
|---|---|
| Brisbane (BR) | G50E, K140I |

a: A Wuhan-like clade which appeared in the northern hemisphere in 96/96.
b: A subclade of the antigenic type California appearing in the summer of 2005.
c: A subclade of the antigenic type Panama.
d: A subclade of the antigenic type Johannesburg
e: Mutations inferred from labeled strains.

FIGs. 4a to 4c shows an exemplary phylogenetic tree built using method 100 and/or method 200. A suitable computer environment for building these trees may be computer system 305 of FIG. 3.

Each of FIGs. 4a to 4c shows the same phylogeny for 1813 isolate sequences of the H3 hemagglutinin precursor gene sampled between 1993 and 2007. Nodes correspond to individual sequences. The node size reflects the number of direct descendants. Nodes are labeled by geographic sampling location (FIG. 4a), season of sampling (FIG. 4b) and antigenic clades (FIG. 4c). For the sake of clarity, merely some of the nodes of the tree are labeled. The skilled person, however, will understand that each isolate node of FIG. 4a may be assigned a geographic sampling location, each isolate node of FIG. 4b may be assigned a season of sampling and each isolate node of FIG. 4c may be assigned an antigenic signature clade. Seasons start with 1992, corresponding to the 92/93 winter season in the northern hemisphere.

An H3 phylogeny according to method 100 was constructed from 1813 dated genetic sequences of the HA0 precursor protein of influenza subtype H3N2. The data were obtained from the NCBI influenza virus resource. They comprise isolates from 35 countries and span the time period from 01/16/1993 (92/93) season in northern hemisphere) to 07/03/2007 (2007 season in the southern hemisphere). 60% of the sequences originate from the northern hemisphere (mostly the United States), 36% from the southern hemisphere (mostly New Zealand and Australia), and 4% from the tropics (mostly from countries in south-east Asia; 80%). Sequences were aligned with ClustalW, version 1.93 using default settings. A nucleotide distance matrix was computed in which distances reflect the number of distinct positions between each pair of sequences in the alignment with gaps being considered a fifth character.

A phylogeny corresponds to a tree built in accordance with method 100. The root node represents the isolate with the earliest sampling date/time stamp. In accordance with method 100, for each node, outgoing edges were added to all nodes with a later sampling date/time stamp.

The phylogeny with the least overall cost (weight) in genetic distance is a rooted minimum spanning tree in this graph as discussed above with respect to method 100. For the trees of FIGs. 4a to 4c, the Chu-Liu/Edmonds algorithm was used and a minimum spanning tree was constructed by selecting the incoming edge with the lowest cost (weight) for each node. In case of multiple incoming edges with identical cost, the edge with the earliest sampling date/ time stamp for the associated source node was chosen. As the input graph is acyclic, no cycles need to be resolved.

The resulting phylogeny of H3 hemagglutinin evolution for the time period from 1993 to 2007 is shown in FIGs. 4a to 4c.

As also described with respect to FIG. 1, a phylogenetic tree may be inferred as follows:

The input to the method is a plurality of sequences with associated sampling dates/time stamps and isolate identifiers.

In an initializing step, for each set of identical sequences among the input sequences, all but one sequence corresponding to the one with the earliest sampling date/time stamp are set aside. The retained sequence may also be the sequence with the latest sampling date/time stamp or a sequence chosen by any other criterion from a set of identical sequences. This results in a sequence set without any duplicated sequences. For the plurality of non-identical sequences an initial optimum branching is computed.

In an update step, the sequences of unsampled evolutionary intermediates are inferred based on differences in sequence between nodes connected by an edge in the optimum branching. The inferred sequences are then added with time stamps to the sequence set. Then, for sets of identical sequences, all but one instance, which may be the instance with the earliest time stamp, are removed. Then, an optimum branching is recomputed. This step is repeated until no new intermediates are found.

In a final step, the sequences that have been set aside in the initializing step are added to the sequence set derived in the update step. From this plurality of sequences an optimum branching may be computed and may be identified as the phylogenetic tree.

Unsampled evolutionary intermediates can be inferred from sequence differences between the sequences associated with the nodes in the minimum spanning tree. FIG. 6 shows exemplary how to infer a phylogeny and the sequences of unsampled evolutionary intermediates from sequence differences in the minimum spanning tree. The middle panel represents the true phylogeny for five isolates. The associated mutation events $x_1$ to $x_3$ are shown on the edges of the tree. As an example, $x_1$ may represent a difference at position 183 between the source (A at position 183 for the source) and sink isolate sequences (G at position 183 for the sink), denoted by A183G. $-x_1$ in this case denotes the reverse of the change, G183A, with G at position 183 of the source node sequence and A at position 183 of the sink node sequence. In the absence of a sequence representing the evolutionary intermediate B among a plurality of analyzed sequences, a minimum spanning tree of the sampled sequences A, C, D and E is represented by the tree topologies on the left and right side of the panel. The associated mutations $x_1$ to $x_3$ are shown on the edges of the trees. Which of these tree topologies is obtained depends on how the least-cost incoming edge is selected in case of multiple incoming least cost edges for a node. In this exemplary case, the choice of the edge may be random, based on minimum or maximum difference in time between the associated sampling dates/time stamps or in some other fashion. Given one of the shown topologies, the intermediate sequence B can be inferred from the mutation patterns on the in- and/or outgoing edges of a node as follows:

In the event of sets of mutations occurring repeatedly on more than one outgoing edge of a node (left part of figure), these are used to infer the sequences of unsampled intermediates by the following procedure: In the example on the left, there is one set which contains mutation $x_1$. If multiple sets exist, for each pair of mutation sets $X_i$ and $X_j$, it is determined whether the relative complements $X_i \setminus X_j$ and $X_j \setminus X_i$ are empty. If for any pair of mutation sets both relative complements are non empty, the node is flagged as "ambiguous". For ambiguous nodes, intermediates are processed in the inference step when no unambiguous nodes remain. Then, an intermediate sequence is created by introducing the changes specified by the largest mutation set into the sequence associated with the parent node.

If a node is not flagged ambiguous, for each mutation set, an intermediate sequence is created. This may be done by introducing the changes specified by the mutation set into a copy of the sequence associated with the parent node. In the example on the left, an intermediate sequence may be derived by introducing mutation $x_1$ into a copy of the sequence of node A. Alternatively, the intermediate sequence may also be derived from the sequence of a descendant node such as C by introducing the reverse of all mutations occurring on the edge of isolate A to C and not contained in the mutation set. The intermediate is then added to the sequence set with a sampling date/time stamp. The sampling date/time stamp is chosen from the time span after the sampling date/time stamp of node A and before the sampling dates/time stamps of the descendant nodes (C, D and E in the example on the left). If a mutation set specifies a subset of the mutations contained in one or more other sets, the sampling date/time stamp for the inferred sequence is chosen from the time span after the sampling dates/time stamps of sequences inferred from these other sets and before the sampling dates/time stamps of the descendant nodes.

The second mutation pattern which may be used to infer the sequence of an unsampled intermediate is a set of (one or more) mutations which occur in reverse order on the in- and outgoing edges of a node, as shown for node C in the example on the right. In this case, the unsampled sequence of the intermediate B may be created from a copy of the sequence associated with node A by introducing all changes on the outgoing edge of A to C which are not reversed on the outgoing edges of C. Alternatively, the sequence associated with node B may be created from a copy of the sequence associated with node C by introducing the reverse of the set of mutations. The new intermediate sequence B is added with a time stamp from the time span after the sampling date/time stamp of node A and before the sampling date/time stamp of node C to the set of sequences.

In the case that different sets of mutations are reversed on different outgoing edges of a node relative to the incoming one, it is first checked for each pair of mutation sets whether the relative complement of both sets is non-empty. If this is case, the node is masked (no intermediates are created based on the patterns at this node) until the remainder of the tree is stable and then an intermediate sequence is created by introducing the changes specified by the largest set of reversed mutations. If this is not the case, for each set of mutations an intermediate sequence is created as described above. The new intermediate sequences are added with time stamps from the time span after the sampling date/time stamp of node A and before the sampling date/time stamp of node C to the set of sequences, in an order according to the number of introduced mutations, such that the intermediate with the maximum number of introduced changes is assigned the earliest time stamp and that no intermediate with a given number of introduced changes has a later time stamp than another intermediate with less changes introduced.

Table 2: Comparison of tree cost (# mutations) and runtime for trees inferred using mutation patterns (PPhylo) and bifurcating maximum parsimony trees computed with PAUP for 13 different sequence sets for influenza subtype H3N2 obtained from the Influenza Sequence Database at NCBI, the number of sequences in a particular set is given in brackets. HA0 refers to the complete sequence of the hemagglutinin precursor, HA1 to subunit 1 of the protein. Sequence sets 3-13 represent sets of all other viral proteins of the virus with the exception of PB1-F2. Runtimes are given in seconds.

| Data set (# sequences) | PPhylo | PAUP | PPhylo | PPhylo | PAUP |
|---|---|---|---|---|---|
| | cost | cost | Extra cost (%) | Runtime (s) | Runtime (s) |
| 1. HA0, year of 1993 (38) | 87 | 87 | 0.00% | 12 | 0.18 |
| 2. HA1, 1993-2007 (1813) | 1913 | 1897 | 0.84% | 6749 | 421755 |
| 3. HA1, 1993-2007 (3142) | 3737 | 3705 | 0.86% | 33638 | 4148239 |
| 4. HA0, 1993-2007 (1366) | 2180 | 2166 | 0.65% | 9163 | 195611 |
| 3. NA, 1993-2007 (1366) | 2013 | 1989 | 1.21% | 4209 | 248995 |
| 6. M1, 1993-2007 (1366) | 538 | 535 | 0.56% | 400 | 416706 |
| 7. M2, 1993-2007 (1366) | 400 | 390 | 2.56% | 84 | 1055683 |
| 8. NP, 1993-2007 (1366) | 1512 | 1494 | 1.20% | 5292 | 175742 |
| 9. NS1, 1993-2007 (1366) | 662 | 655 | 1.07% | 561 | 495710 |
| 10. NS2, 1993-2007 (1366) | 354 | 345 | 2.61% | 115 | 1474474 |
| 11. PA, 1993-2007 (1366) | 2131 | 2115 | 0.76% | 9627 | 122390 |
| 12. PB1, 1993-2007 (1366) | 2375 | 2362 | 0.55% | 13309 | 86257 |
| 13. PB2, 1993-2007 (1366) | 2271 | 2254 | 0.75% | 9268 | 100706 |

The cost in number of mutations and run time on a Dell Poweredge 6950 (4 x Opteron 8220 2,8 GHz 2 x 1 MB, Dual Core AMD Opteron Processor) for phylogenetic trees computed with the described method with thirteen sequence sets of different proteins of influenza, subtype H3N2 were compare to maximum parsimony trees computed with PAUP (version 4.0b10 for UNIX). PAUP* was used with the default settings except the following options. The sequences were handled as datatype='STANDARD' to disable usual DNA macros. Gaps were treated as additional state (GAPMODE=NEWSTATE), i.e. as a fifth base. The trees were rooted with the oldest sequence. The maximum number of stored trees was set to 200 and was not further increased. The optimization criterion was set to 'parsimony' and the tree space was searched by heuristic search, where the sequences were randomly added within the tree building procedure for the initial tree. Table 2 shows the mutation cost and runtimes for the different data sets; the described method is 10-1000 times faster and generates trees with an extra cost of 0 - 2.61 % in terms of mutations.

The phylogenetic tree can then be used to analyze the development of antigenic lineages (FIG. 4c), to identify sites under positive selection, amino acid mutations associated with antigenic drift and viral lineages with the potential to become predominant in the next winter season, as well as the geographic spread and origin of successful strains (FIG. 4a).

Antigenic lineages: For visualizing the antigenic lineages or antigenic clades, in a first step, the node sequences are assigned based on the presence of signature mutations (Table 1). If one isolate matches multiple signatures, the isolate is assigned to the latest occurring type (e.g. isolates of the type Panama can still carry the signature mutations of the previous type Sydney, as the characteristic positions are distinct). If a descendant of a signature-matching isolate acquired a change in one of the signature sites that does not match a subsequently appearing signature, it is left unassigned. In the example analysis 92% (1673) of the isolates could thus be assigned.

Visualization of the antigenic assignments reveals a consistent clade structure (FIG. 4c). Isolates in a clade are antigenically similar but not necessarily identical. According to WHO reports, in particular in the late stages of circulating types, variation in antigenicity is observed. To elucidate phenotypic properties in detail, experimental tests of viral isolates with techniques such as the hemagglutination inhibition assay can be performed, and the similarity can be revealed with known techniques such as multidimensional scaling [F. J. Poelwijk, et al., Nature 445 (7126), 383 (2007)].

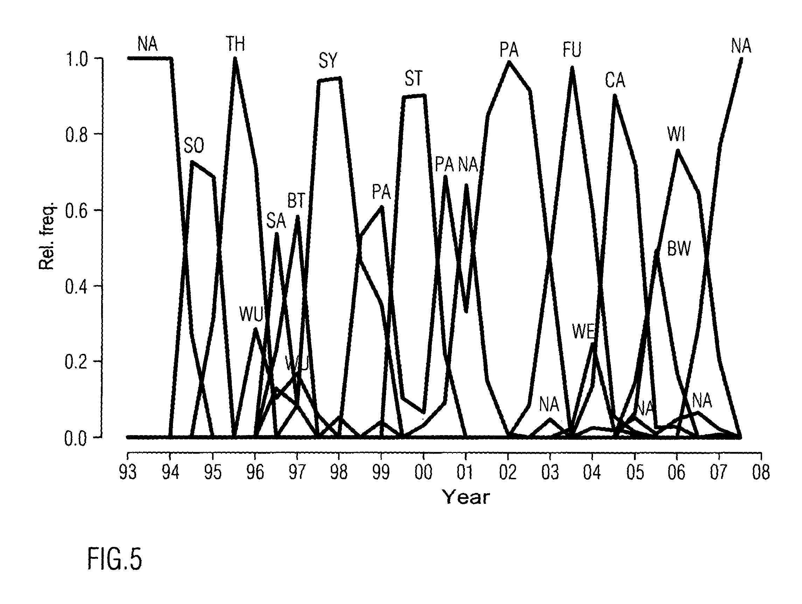

For all clades, the timing of prevalence shown in the tree of FIGS. 4a to 4c agrees with epidemiological reports (FIG. 5). Types (named after vaccine strains) appear as predominant in the following order:

Johannesburg (1994-1995) and the Johannesburg-like clade Thessaloniki (1995-1996), Wuhan and the Wuhan-like clade Bratislava (1996-1997), Sydney (1997-1998), Panama (1998-2003) and the clade Stockholm (1999-2000), Fujian (2002-2004), California (2004-2005) and the California-like clade Baden-Wuerttemberg (2005-2006), Wisconsin (2005-2007) and Brisbane (clade I-3 of Wisconsin, 2006-2007). The reported signature mutations can therefore reveal the antigenic clade structure of the tree and allow to study their properties in the phylogeny.

Sites under positive selection: The exemplary influenza phylogeny contains 1915 nucleotide and 926 amino acid mutations. The majority of amino acid mutations occur at the tips of the tree, 718 nucleotide and 344 amino acid changes are located on internal branches. Changes at hemagglutinin sites under positive selection possess predictive value for the identification of progenitors for future (e.g. H3) lineages. Since 1999, the data has more than quadrupled, which allows the identification of novel sites of importance. These novel sites of importance are characterized by a significant excess of nonsynonymous over synonymous mutations on internal branches of the phylogeny.

A synonymous mutation is defined as a mutation in which one nucleotide is substituted for another, but the encoded amino acid remains unchanged (i.e., UUA to UUG, both specifying leucine). A nonsynonymous mutation is defined as nucleotide substitution that changes the amino acid specified (i.e., AGC to AGA, or serine to arginine). A nonsynonymous substitution is equivalent to a nonsynonymous mutation or amino acid mutation or amino acid change.

Sites under positive selection were identified as described [R. M. Bush, et al., Mol. Biol. Evol. VOL 16 NO 11, page 1457 (1999)], based on a significant excess of nonsynonymous over synonymous mutations on internal branches of the phylogeny. The probability for a nonsynonymous substitution to occur under the null model of no positive selection was calculated from the phylogeny based on the relative frequency of nonsynonymous mutations among mutations in non-epitope regions of the protein. The significance of an observed ratio of synonymous to nonsynonymous mutations on internal branches of the phylogeny at a particular site was calculated from the binomial distribution. The multiple testing error was corrected with the false discovery rate [Y. and Hochberg Benjamini, Y., Journal of the Royal Statistical Society VOL 57, NO 289 (1995)].

16 such sites under positive selection were identified in the HA1 subunit sequences (with p-values < 0.05),. 11 of the sites are in or close to the epitope regions of HA1, and most are exposed on the protein surface (Table 3).

Table 3: Sequence positions in HA1 subunit under positive selection identified by significant enrichment of non-synonymous substitutions on internal branches of the phylogeny.

| Site | Nsyn. | Syn. | Antigenic drift residue [d] | Antigenic site [a] | Bush et al. site [3] | % Surface exposure [b] |
|------|-------|------|---------------------|---------------|------------------|-------------------|
| 45 | 6 | 0 | | | | 13.6413 |
| 49 | 4 | 0 | | | | 8.2949 |
| 50 | 10 | 1 | | x | | 25.9763 |
| 53 | 5 | 0 | C | x | | 15.9248 |
| 121 | 4 | 0 | | x | X | 13.9794 |
| 138 | 4 | 0 | | | X | 3.5439 |
| 142 | 7 | 1 | | x | X | 18.2336 |
| 144 | 7 | 0 | A | | | 7.3392 |
| 145 | 9 | 0 | A | x | X | 16.0612 |
| 173 | 4 | 0 | | x | | 36.0493 |
| 186 | 5 | 1 | B | x | x | 5.3243 |
| 199 | 5 | 1 | | x | | 23.7655 |
| 220 | 6 | 0 | D | x | | 0 |
| 226 | 10 | 6 | X | x | X | 12.7562 |
| 229 | 6 | 1 | | | | 4.1469 |
| 275 | 8 | 1 | C | x | X | 16.8292 |

[a]: 89 sites in antigenic regions defined in [D. C. Wiley, et al., Nature 289 (5796),373 (1981)] with more than 7% surface exposure of residues except for positions in the subunit interface region (site D). [b]: Accessibility computed with method at http://swift.cmbi.ru.nl/servers/html/index.html based on PDB structure 2HMG. [c]: Part of receptor binding site. [d]: Described in [D. C. Wiley, et al., Nature 289 (5796),373 (1981) and C. A. Smith, et al., J Exp Med 173(4), 953 (1991).

The sites include seven of the eighteen sites identified from earlier data (1983-1997: 121, 138, 142, 145, 186, 226 and 275), which implies their continuous importance in antigenic drift. Some of the other previously found sites still feature an excess of nonsynonymous to synonymous mutations, but the total number of changes is insufficient for significance. The newly identified sites were utilized more strongly in antigenic drift recently than in the previous time period. Thus, there are commonalities, but also some variation in the sites most relevant for antigenic drift during different periods of time. By including more data, the picture of the evolutionary space for antigenic drift can become even more complete.

Repeatedly occurring amino acid mutations associated with antigenic drift and predominant strains of the following season: The phylogeny represents a succession of evolutionary accessible viral variants in the evolution of novel antigenic types. Experimental studies on enzymes can map intermediates in the evolution of a suboptimal allele towards an optimal version on phenotypic fitness landscapes [F. J. Poelwijk, et al., Nature 445 (7126), 383 (2007)]. It has been found that the local landscapes are single peaked (i.e. there is one optimum). There are many unlikely paths on which intermediates show a decrease or no change in fitness and a few favorable ones, which are accessible to Darwinian selection due to successive fitness increases with each introduced mutation. If there is a fitness increase associated with a particular mutation, it occurs more frequently than expected among isolates in that part of the tree. That is, favorable mutations, or mutations that imply a fitness increase appear more often in the phylogenetic tree, while mutations that decrease the fitness occur less often.

The exemplary phylogenetic tree of 1813 isolate sequences of the HA1 subunit of the influenza hemagglutinin contains 1915 mutation events, corresponding to 926 distinct amino acid mutations. 195 amino acid mutations appear multiple times in different lineages of the tree, at 125 sites of the HA1 sequence. These sites are enriched in epitope sites and sites under positive selection ($p$-value=$3.34^{-10}$ and $5.32^{-7}$ by hypergeometric distribution), stressing their importance in antigenic drift.

For the amino acid mutations occurring multiple times on edges in the phylogeny, it is important to identify their localization pattern in the phylogeny (i.e. in the tree) and to determine whether they cluster in the tree. This analysis of the clustering behaviour of repeated mutations is performed as follows: The null model is that all mutations in the phylogeny are equally beneficial and there is no significant difference in the path length between the nodes with the same mutation on the incoming edge in the tree in comparison to nodes with other mutations on the incoming edges. It is to determine whether this assumption can explain the observed average path length between nodes with identical mutations. For significance estimation the mutation environment for a set of nodes with the same mutation on the incoming edges of size n is defined as follows: The mutation environment is the distribution over the average path lengths between one of these nodes and n-1 nodes with different mutations on the incoming edges. For each mutation occurring multiple times, the mutation environment distribution was calculated by randomly sampling sets of size n containing one of the n nodes and n-1 nodes with different mutations on the incoming edges from the phylogeny, and computing the average path lengths between all pairs of nodes in the set. Sampling was done 10,000 times. A mutation environment was calculated for each mutation occurring on multiple edges individually as the environment may differ in different parts of the tree, due to the sampling imbalance of the data set in different seasons and geographic locations. From each mutation environment distribution a *p*-value was obtained for the corresponding set of nodes with the respective mutation, reflecting the significance of their clustering within the phylogeny. The *p*-value obtained for every multiple occurring mutation from the corresponding mutation environment distribution was corrected for multiple testing with the false discovery rate. Of the 195 mutations occurring multiple times, 140 the corresponding nodes are clustered with the average path length between all pairs of nodes being shorter than the mean of the corresponding mutation environment distribution.

The clustered mutations are enriched in antigenic signature mutations; 27 of the 50 signature mutations are represented (Table 4). In contrast, only one signature change is among the non-clustered repeated mutations, respectively.

Table 4: Isolates featuring repeated amino acid mutations with duster in the phylogeny and match signature mutations of antigenic types. For each isolate, the amino acid mutation, corresponding antigenic signature, number of independent occurrences in the phylogeny, antigenic clade and sampling date of isolate is given.

| Mutation | Signature clade | Counts | Isolate clade | Sampling date |
|---|---|---|---|---|
| (a) | | | | |
| D124N | JO | 2 | JO | 18/07/1993 |
| D124N | JO | 2 | NA | 17/01/1994 |
| S219Y | JO | 6 | JO | 18/07/1993 |
| S219Y | JO | 6 | FU | 25/12/2002 |
| S219Y | JO | 6 | PA | 6/2/2003 |
| S219Y | JO | 6 | FU | 20/06/2003 |
| S219Y | JO | 6 | FU | 16/08/2003 |
| S219Y | JO | 6 | CA | 16/05/2004 |
| N145K | WU | 4 | NA | 19/04/1993 |
| N145K | WU | 4 | WU | 6/12/1994 |
| N145K | WU | 4 | JO | 25/03/1995 |
| N145K | WU | 4 | TH | 3/1/1996 |
| N262S | WU | 3 | NA | 26/03/1993 |
| N262S | WU | 3 | NA | 14/01/1994 |
| N262S | WU | 3 | WU | 6/12/1994 |
| G275D | BT | 11 | TH | 30/07/1994 |
| G275D | BT | 11 | TH | 10/1/1995 |
| G275D | BT | 11 | WU | 16/07/1996 |
| G275D | BT | 11 | SA | 14/08/1996 |
| G275D | BT | 11 | SA | 13/09/1996 |

(continued)

| Mutation | Signature clade | Counts | Isolate clade | Sampling date |
|---|---|---|---|---|
| G275D | BT | 11 | PA | 18/10/2001 |
| G275D | BT | 11 | FU | 4/3/2003 |
| G275D | BT | 11 | CA | 27/10/2004 |
| G275D | BT | 11 | CA | 27/06/2005 |
| G275D | BT | 11 | WI | 19/10/2006 |
| G275D | BT | 11 | CA | 5/3/2007 |
| R57Q | BT | 3 | WU | 31/08/1996 |
| R57Q | BT | 3 | SA | 15/11/1996 |
| R57Q | BT | 3 | SY | 19/03/1998 |
| D133N | SA | 2 | SA | 21/01/1996 |
| D133N | SA | 2 | WU | 6/1/1997 |
| G124S | SA | 2 | TH | 7/2/1995 |
| G124S | SA | 2 | SA | 21/01/1996 |
| E158K | SY | 2 | NA | 3/11/1994 |
| E158K | SY | 2 | SY | 8/1/1997 |
| K156Q | SY | 3 | NA | 3/11/1994 |
| K156Q | SY | 3 | JO | 21/04/1995 |
| K1564 | SY | 3 | SY | 8/1/1997 |
| V196A | SY | 3 | NA | 29/07/1994 |
| V196A | SY | 3 | NA | 3/11/1994 |
| V196A | SY | 3 | SY | 8/1/1997 |
| N1441 | ST | 2 | ST | 9/6/1999 |
| N1441 | ST | 2 | ST | 12/2/2000 |
| Q156H | FU | 3 | FU | 25/04/2001 |
| Q156H | FU | 3 | FU | 7/6/2002 |
| Q156H | FU | 3 | FU | 29/11/2002 |
| S189N | WE | 5 | PA | 11/9/2000 |
| S189N | WE | 5 | PA | 31/12/2001 |
| S189N | WE | 5 | FU | 28/05/2003 |
| S189N | WE | 5 | FU | 7/7/2003 |
| S189N | WE | 5 | FU | 24/07/2003 |
| Y159F | WE | 3 | FU | 22/04/2003 |
| Y159F | WE | 3 | WE | 13/10/2003 |
| Y159F | WE | 3 | FU | 27/11/2003 |
| V226I | CA | 16 | SA | 18/12/1996 |
| V226I | CA | 16 | SA | 18/12/1996 |
| V226I | CA | 16 | SY | 29/06/1997 |
| V226I | CA | 16 | ST | 21/09/2000 |

(continued)

| Mutation | Signature clade | Counts | Isolate clade | Sampling date |
|----------|-----------------|--------|---------------|---------------|
| V226I | CA | 16 | PA | 30/06/2001 |
| V226I | CA | 16 | PA | 2/5/2002 |
| V226I | CA | 16 | FU | 18/10/2002 |
| V226I | CA | 16 | FU | 25/12/2002 |
| V226I | CA | 16 | NA | 18/03/2003 |
| V226I | CA | 16 | FU | 24/06/2003 |
| V226I | CA | 16 | FU | 10/8/2003 |
| V226I | CA | 16 | FU | 23/12/2003 |
| V226I | CA | 16 | CA | 6/1 /2004 |
| V226I | CA | 16 | CA | 8/1/2004 |
| V226I | CA | 16 | FU | 20/01/2004 |
| V226I | CA | 16 | CA | 6/9/2004 |
| K173E | BW | 6 | SY | 29/12/1997 |
| K173E | BW | 6 | ST | 24/12/1999 |
| K173E | BW | 6 | CA | 8/2/2004 |
| K173E | BW | 6 | FU | 22/04/2004 |
| K173E | BW | 6 | WI | 13/02/2006 |
| K173E | BW | 6 | BR | 21/01/2007 |
| V112I | BW | 5 | WU | 1/4/1996 |
| V112I | BW | 5 | NA | 26/11/2002 |
| V112I | BW | 5 | PA | 6/3/2003 |
| V112I | BW | 5 | FU | 22/04/2003 |
| V112I | BW | 5 | CA | 12/3/2004 |
| G50E | BR | 8 | FU | 7/8/2003 |
| G50E | BR | 8 | FU | 16/08/2003 |
| G50E | BR | 8 | FU | 22/09/2003 |
| G50E | BR | 8 | FU | 29/09/2003 |
| G50E | BR | 8 | FU | 29/12/2003 |
| G50E | BR | 8 | WE | 5/8/2004 |
| G50E | BR | 8 | WI | 17/04/2005 |
| G50E | BR | 8 | BR | 23/10/2006 |
| D124N | JO | 2 | JO | 18/07/1993 |
| D124N | JO | 2 | NA | 17/01/1994 |
| S219Y | JO | 6 | JO | 18/07/1993 |
| S219Y | JO | 6 | FU | 25/12/2002 |
| S219Y | JO | 6 | PA | 6/2/2003 |
| S219Y | JO | 6 | FU | 20/06/2003 |
| S219Y | JO | 6 | FU | 16/08/2003 |

(continued)

| Mutation | Signature clade | Counts | Isolate clade | Sampling date |
|---|---|---|---|---|
| S219Y | JO | 6 | CA | 16/05/2004 |
| N145K | WU | 4 | NA | 19/04/1993 |
| N145K | WU | 4 | WU | 6/12/1994 |
| N145K | WU | 4 | JO | 25/03/1995 |
| N145K | WU | 4 | TH | 3/1/1996 |
| N262S | WU | 3 | NA | 26/03/1993 |
| N262S | WU | 3 | NA | 14/01/1994 |
| N262S | WU | 3 | WU | 6/12/1994 |
| G275D | BT | 11 | TH | 30/07/1994 |
| G275D | BT | 11 | TH | 10/1/1995 |
| G275D | BT | 11 | WU | 16/07/1996 |
| G275D | BT | 11 | SA | 14/08/1996 |
| G275D | BT | 11 | SA | 13/09/1996 |
| G275D | BT | 11 | PA | 18/10/2001 |
| G275D | BT | 11 | FU | 4/3/2003 |
| G275D | BT | 11 | CA | 27/10/2004 |
| G275D | BT | 11 | CA | 27/06/2005 |
| G275D | BT | 11 | WI | 19/10/2006 |
| G275D | | | | |
| R57Q | BT | 3 | W U | 31/08/1996 |
| R57Q | BT | 3 | SA | 15/11/1996 |
| R57Q | BT | 3 | SY | 19/03/1998 |
| D133N | SA | 2 | SA | 21/01/1996 |
| D133N | SA | 2 | WU | 6/1/1997 |
| G124S | SA | 2 | TH | 7/2/1995 |
| G124S | SA | 2 | SA | 21/01/1996 |
| E158K | SY | 2 | NA | 3/11/1994 |
| E158K | SY | 2 | SY | 8/1/1997 |
| K156Q | SY | 3 | NA | 3/11/1994 |
| K156Q | SY | 3 | JO | 21/04/1995 |
| K156Q | SY | 3 | SY | 8/1/1997 |
| V196A | SY | 3 | NA | 29/07/1994 |
| V196A | SY | 3 | NA | 3/11/1994 |
| V196A | SY | 3 | SY | 8/1/1997 |
| N144I | ST | 2 | ST | 9/6/1999 |
| N144I | ST | 2 | ST | 12/2/2000 |
| Q156H | FU | 3 | FU | 25/04/2001 |
| Q156H | FU | 3 | FU | 7/6/2002 |

(continued)

| Mutation | Signature clade | Counts | Isolate clade | Sampling date |
|----------|-----------------|--------|---------------|---------------|
| Q156H | FU | 3 | FU | 29/11/2002 |
| S189N | WE | 5 | PA | 11/9/2000 |
| S189N | WE | 5 | PA | 31/12/2001 |
| S189N | WE | 5 | FU | 28/05/2003 |
| S189N | WE | 5 | FU | 7/7/2003 |
| S189N | WE | 5 | FU | 24/07/2003 |
| Y159F | WE | 3 | FU | 22/04/2003 |
| Y159F | WE | 3 | WE | 13/10/2003 |
| Y159F | WE | 3 | FU | 27/11/2003 |
| V226I | CA | 16 | SA | 18/12/1996 |
| V226I | CA | 16 | SA | 18/12/1996 |
| V226I | CA | 16 | SY | 29/06/1997 |
| V226I | CA | 16 | ST | 21/09/2000 |
| V226I | CA | 16 | PA | 30/06/2001 |
| V226I | CA | 16 | PA | 2/5/2002 |
| V226I | CA | 16 | FU | 18/10/2002 |
| V226I | CA | 16 | FU | 25/12/2002 |
| V226I | CA | 16 | NA | 18/03/2003 |
| V226I | CA | 16 | FU | 24/06/2003 |
| V226I | CA | 16 | FU | 10/8/2003 |
| V226I | CA | 16 | FU | 23/12/2003 |
| V226I | CA | 16 | CA | 6/1 /2004 |
| V226I | CA | 16 | CA | 8/1/2004 |
| V226I | CA | 16 | FU | 20/01/2004 |
| V226I | CA | 16 | CA | 6/9/2004 |
| K173E | BW | 6 | SY | 29/12/1997 |
| K173E | BW | 6 | ST | 24/12/1999 |
| K173E | BW | 6 | CA | 8/2/2004 |
| K173E | BW | 6 | FU | 22/04/2004 |
| K173E | BW | 6 | WI | 13/02/2006 |
| K173E | BW | 6 | BR | 21/01/2007 |
| V112I | BW | 5 | WU | 1/4/1996 |
| V112I | BW | 5 | NA | 26/11/2002 |
| V112I | BW | 5 | PA | 6/3/2003 |
| V112I | BW | 5 | FU | 22/04/2003 |
| V112I | BW | 5 | CA | 12/3/2004 |
| G50E | BR | 8 | FU | 7/8/2003 |
| G50E | BR | 8 | FU | 16/08/2003 |

(continued)

| Mutation | Signature clade | Counts | Isolate clade | Sampling date |
|---|---|---|---|---|
| G50E | BR | 8 | FU | 22/09/2003 |
| G50E | BR | 8 | FU | 29/09/2003 |
| G50E | BR | 8 | FU | 29/12/2003 |
| G50E | BR | 8 | WE | 5/8/2004 |
| G50E | BR | 8 | WI | 17/04/2005 |
| G50E | BR | 8 | BR | 23/10/2006 |

Many of the clustered amino acid mutations emerge in independent lineages one to three years before establishment of a novel antigenic type. An example of such a mutation is G50E of the Brisbane clade signature. Prior to the establishment of Brisbane, G50E already occurred in other lineages of the Wisconsin clade in 2006, the clade Wellington in 2004 and Fujian in 2003. In the Wisconsin clade, it is accompanied by the K140I mutation, giving rise to clade Brisbane. The California and Wellington signature mutation S189N appeared independently three times in the precursor Fujian in 2003 and twice in the Panama clade, in 2000 and 2001. For a certain window in time, there is a distinct advantage associated with these mutations on the viral fitness landscape; already before they occur in combination with others in a novel and dominating antigenic type.

Geographic spread and origin of successful strains: Seasonal influenza infections predominantly occur in the winter season of the northern and southern hemispheres, but year-round in the tropics. Understanding the epidemiology of the virus requires resolving the details of viral geographic spread. As sampling dates and geographic location are available, visualization of these properties in combination with the antigenic clade structure conveniently reveals information on the timing and geographic prevalence of the successively emerging viral lineages (FIGs. 4).

Implications: For influenza A, the phylogeny identifies (i) repeated amino acid mutations significantly associated with antigenic drift which allow the recommendation of suitable vaccine strains matching the predominant viral phenotype of the upcoming influenza season (ii) the sites most relevant for antigenic drift and (iii) clades corresponding to the dominating antigenic types of the virus during the analyzed time span.

It is therefore apparent that the phylogeny of the present invention is a powerful tool for resolving the evolutionary history of the influenza virus. Thus, a further aspect of the invention relates to a method for the production of a vaccine against a fast-evolving entity by analyzing biological sequences of a plurality of isolates to generate a phylogenetic tree, the recommendation or selection of a suitable individual isolate of the entity as a candidate for the forthcoming virus outbreak season, and the preparation of a vaccine based on such a recommendation or selection.

The recommendation or selection of an individual isolate of the fast-evolving entity comprises the steps of (i) computation of a phylogenetic tree from sequences available up to the point in time where the decision is to be made, (ii) identifying repeated mutations in a phylogenetic tree, (iii) identifying the number of repeated mutations occurring in each isolate and (iv) the selection of an isolate as a vaccine candidate when the isolate bears the maximum number of repeated mutations that only occur on the incoming edges of nodes with timestamps from a time-span of 3 or 4 years before the season in which the vaccine is to be produced and are located within an antigenic site of the hemagglutinin. Specifically, an isolate is selected as a vaccine in step (iv) when (a) the isolate sequence bears the maximum number of repeated amino acid mutations that occurred within a time-span of 4 years before the season in which the vaccine is to be produced, or (b) the isolate sequence bears the maximum number of repeated amino acid mutations that are located in antigenic sites and occurred within a time-span of 4 years before the season in which the vaccine is to be produced, or (c) the isolate sequence bears the maximum number of repeated amino acid mutations that cluster in the phylogeny, are located in antigenic sites and occurred within a time-span of 3 years before the season in which the vaccine is to be produced, or (d) the isolate sequence bears the maximum number of repeated amino acid mutations that cluster in the phylogeny, are located in sites determined experimentally to change the viral phenotype and occurred within a time-span of 4 years before the season in which the vaccine is to be produced.

In another embodiment of this aspect of the invention, the clustering behaviour of amino acid mutations can be taken into account in the selection process. That is, an isolate is selected if when the isolate bears the maximum number of repeated amino acid mutations which (i) cluster with the average path length between all pairs of nodes being shorter than the mean of the corresponding mutation environment distribution calculated as described above, (ii) occur only on the incoming edges of nodes with time stamps from a time-span of 3 or 4 years up to the season in which the vaccine is to be produced and (iii) are located within an antigenic site of the hemagglutinin.

In another embodiment of this aspect of the invention, the geographic spread can be taken into account in the selection process. That is, if a vaccine for the northern hemisphere is desired and more than one otherwise equally suitable isolates from different geographic regions have been identified, the preference is given to the strain which was sampled from the northern hemisphere.

In a further embodiment, the weight of the nodes can be taken into account in the selection process. The weight of a node is determined by the number of its direct progeny, e.g. a "parent" isolate with 20 direct descendants is more suitable as a vaccine than an isolate with only 3 descendants (provided the remaining parameters outlined above are identical).

In a further embodiment the prediction on the basis of the clustered repeated mutations can be combined with the calculation of $P_{epitope}$. $P_{epitope}$ is a variable that measures the antigenic distance between two influenza strains. For a given viral strain, the change in antigenicity relative to a reference strain is related to the genetic change in the antigenic site which exhibits the most change of the viral hemagglutinin surface protein (the dominant antigenic site). $P_{epitope}$ is the normalized difference between the vaccine strain used in a particular season and the analyzed strain within the dominant antigenic site. It was shown that $P_{epitope}$ has a positive correlation with vaccine efficacy [Gupta, V. et al., Vaccine 24(18), 2006:3881-8]. The results of an exemplary analysis of biological isolate sequences are shown in Tables 5 and 6.

Table 5: Repeated mutations used to select isolate sequences as vaccine strain recommendations for seasons in which a vaccine strain update was required. Column 2 list all repeatedly occurring mutations identified from phylogenies constructed from the available HA1 sequences up to the season in which the vaccine strain recommendation is to be made (e.g. with data up to March of 1995 for the 95/96N prediction), which occur only on incoming edges of nodes with a time stamp from within the last four years. In brackets the non-clustered repeated mutations are given. Mutations localizing to the antigenic sites of the HA1 protein are shown in bold font. Mutations or sequence changes given in the table have the format "previous_aminoacid" "sequence_position" "new_aminoacid", e.g. G186V signifies that glycine at position 186 is replaced by valine. Sequences in the test set are scored with the mutation list for the respective season, adding one count for every match of the 'new_aminoacid' at "sequence position" (e.g. for D188Y, the score for a sequence is incremented by one if the tested sequence features a "Y" at position 188).

| Year and hemisphere | Mutations |
|---|---|
| 95/96N | **N246S, (A138S, N145K, N262S)** |
| 98/99N | **K207Q, L164Q,** R220G, **R229I, (R142G, T192I,** V223I) |
| 1999S | D271 N, **R229K, S186G, (K92T, T192I)** |
| 2002S | D291G, I288V, I58V, K2E, R220X, **R229K,** R269K, **T1671,** (T10M) |
| 02/03N | **E62G,** G225D, I267V, **N144T, Q156H**, W222R, **(A272T) G50E,** G78S, I34L, **K145N, K92R, N144D, S189N, S193N, S451, Y159F,** |
| 03/04N | **(D188E,** D77E, L3F, **N312K,** N8K, **V226X) D188N, G186V, G50E, I230M, K145N, K92R, S193N,** S199P, **S262N,**Y105H, |
| 2004S | **Y159F,** (A106V, **A304P, G186S,** T12M) **G186V, G50E, I217V, I67M, N145S, N216S, P227S, S124N, T131N, (N165K,** |
| 2005S | T10R) D225N, **G50E, K276N,** K27R, **K83R, K92R,** L66I, **M168L, N216S,** N225D, |
| 05/06N | Q327K, **S124N,** S199A, **T128A,** Y195H |
| 2006S | **D188G,** D225N, **E50G,** N225D **D188Y, E50G, G186V, G50E, I**34V, **K140I,** K259R, K326R, **L194P,** N225D, |
| 06/07N | **N96K,** P221X, **R261Q, S124N,** Y105H, Y195X |

* Epitope Sites in bold font, N = northern hemisphere, S = southern hemisphere

Table 6: Accuracy of vaccine strain recommendations for seasons in which a vaccine strain update was required. A strain is selected one year before. Column 1 gives the seasons for which a vaccine update was required for the northern hemisphere (N) and southern hemisphere (S), respectively. The number of sequenced isolates from the corresponding season of the preceding year which were available for testing with our method is given in column 4. The predominating strain in the season for which the vaccine was to be produced is shown in column 5, WHO influenza vaccine strain recommendation for the season in column 6, wherein boldface signifies a correct recommendation and italics signifies a wrong recommendation. Columns 7-10 show for the sequences recommended with our method the percentage matching the phenotype-signature mutations of the dominating strain for the season, wherein boldface signifies a correct recommendation and italics signifies a wrong recommendation. In brackets the number of recommended sequences is given. Sequences were scored as described above, based on the number of repeated mutations (rMs, columns 9,10) or clustered repeated mutations (crMs, columns 7,8) which occurred in the four preceding years in a phylogeny built from all HA1 sequences available until the end of the season of the preceding year (e.g. from sequences available until the end of March of 1995 for the 95/96N vaccine strain recommendation). Columns 7 and 9 show the results under consideration of mutations at all HA1 positions, columns 8 and 10 show the results under consideration of mutations within the antigenic sites of the hemagglutinin.

| | | | | | | crMs | crMs | rMs | rMs |
|--------|--------|--------|------|-------------|-----|---------|---------|---------|---------|
| Season | From | To | Test | Predominant | WHO | All | Epitope | All | Epitope |
| 96/97N | Oct-96 | Mar-97 | 11 | WU | **WU** | **1 (2)** | **1 (2)** | **1 (2)** | **1 (2)** |
| 99/00N | Oct-99 | Mar-00 | 47 | PA | *SY* | **0.875 (8)** | **1 (4)** | **1 (9)** | **1 (5)** |
| 03/04N | Oct-03 | Mar-04 | 20 | FU | *PA* | **1 (1)** | **1 (1)** | **1 (1)** | **1 (1)** |
| 04/05N | Oct-04 | Mar-05 | 70 | CA | *FU* | **0.5 (2)** | **0.5 (2)** | **0.5 (2)** | **0.5 (2)** |
| 06/07N | Oct-06 | Mar-07 | 68 | WI | **WI** | **1 (2)** | **1 (1)** | **1 (2)** | **1 (1)** |
| 07/08N | Oct-07 | Mar-08 | 77 | BR | *WI* | **1 (1)** | **1 (8)** | **1 (1)** | **1 (8)** |
| | | | | | | | | | |
| 2000S | Apr-00 | Sep-00 | 5 | PA | **PA** | *0 (1)* | **0.5 (2)** | *0 (1)* | *0 (1)* |
| 2003S | Apr-03 | Sep-03 | 46 | FU | *PA* | *0 (1)* | *0 (4)* | *0 (1)* | *0 (4)* |
| 2005S | Apr-05 | Sep-05 | 44 | CA | *WE* | **1 (1)** | **1 (1)** | **1 (1)** | **1 (1)** |
| 2006S | Apr-06 | Sep-06 | 47 | WI | *CA* | *0 (1)* | *0 (1)* | *0 (1)* | *0 (1)* |
| 2007S | Apr-07 | Sep-07 | 4 | BR | *WI* | *0 (2)* | *0 (2)* | *0 (2)* | *0 (2)* |
| Accuracy | | | | | 0.27 | 0.58 | 0.64 | 0.59 | 0.59 |

Antigenic sites correspond to those defined in Wilson and Cox (1990), Structural Basis of Immune Recognition of Influenza Virus Hemagglutinin, Annual Review of Immunology, VOL 8. pages 737-771.

In a further embodiment, the fast-evolving entity selected by the inventive methods can be used for the preparation of a vaccine.

While the invention has been described with respect to the physical embodiments constructed in accordance therewith, it will be apparent to those skilled in the art that various modifications, variations and improvements of the present invention may be made in the light of the above teachings and within the purview of the appended claims without departing from the spirit and intended scope of the invention. In addition, those areas in which it is believed that those of ordinary skill in the art are familiar, have not been described herein in order to not unnecessarily obscure the invention described herein. Accordingly, it is to be understood that the invention is not to be limited by the specific illustrative embodiments, but only by the scope of the appended claims.

**Claims**

1.    A method for identifying a phylogenetic tree for a plurality of biological sequences, each biological sequence being associated with a time stamp, the method comprising:

obtaining distances between biological sequences of the plurality of biological sequences;
generating (120) a graph representation of the plurality of biological sequences based on the distances and the time stamps associated with the plurality of biological sequences;
identifying (125) a minimum spanning tree, MST, of the generated graph as the phylogenetic tree.

2. The method of claim 1, wherein generating the graph representation comprises:

(i) determining identical sequences in the plurality of biological sequences;
(ii) for each set of identical sequences, selecting a biological sequence from the set of identical sequences associated with the earliest time stamp;
(iii) selecting all unique biological sequences;
(iv) creating (206) for each selected biological sequence of the plurality of biological sequences a node representing the selected biological sequence, each node being associated (208) with the time stamp of the respective selected biological sequence; and
(v) adding (222, 244) an outgoing edge from a first node to a second node, if the time stamp associated with the second node is later than the time stamp associated with the first node.

3. The method of claim 2, wherein adding comprises:

determining (216) the node representing the biological sequence associated with the earliest time stamp; and
adding (222) outgoing edges from the determined node to all other nodes.

4. The method of claim 2 or 3, further comprising weighting (224, 246) each one of the added edges with a distance between the biological sequences represented by the nodes connected by the edge, the distance being derived from a distance matrix and wherein identifying the MST comprises:

selecting for each node the incoming edge with the lowest weight; and
removing all edges not selected.

5. The method of one of claims 2 to 4, further comprises:

(vi) identifying an initial MST from the generated graph;
(vii) inferring sequences of unsampled evolutionary intermediates based on differences between sequences represented by nodes connected by an edge in the initial MST;
(viii) assigning time stamps to the inferred sequences of unsampled evolutionary intermediates;
(ix) adding the inferred sequences to the plurality of biological sequences;
(x) repeating steps (i) to (ix) until no new unsampled evolutionary intermediates are inferred;
(xi) adding the sequences not selected at the iterations of step (ii) to the set of biological sequences being selected after step (x);
(xii) creating for each biological sequence of the set of biological sequences derived at step (xi) a node representing the biological sequence, each node being associated with the time stamp of the respective biological sequence; and
(xiii) adding an outgoing edge from a first node to a second node, if the time stamp associated with the second node is later than the time stamp associated with the first node.and

wherein the MST identified as the phylogenetic tree is the MST identified in the graph generated at step (xiii).

6. The method of one of claims 2 to 5, further comprising:

if the time stamps associated with the plurality of biological sequences are specified with a resolution of a time span, connecting all nodes representing biological sequences associated with sampling dates within the same time span; and
resolving cycles in the generated graph during identification of the MST.

7. A system for identifying a phylogenetic tree for a plurality of biological sequences, each biological sequence being associated with a time stamp, the system comprising:

a data preparer (315) adapted to obtain and output distances between biological sequences of the plurality of biological sequences; and
a phylogeny identifier (335) communicatively coupled to the data preparer, the phylogeny identifier comprises:

a graph generator (340) adapted to generate a graph representation of the plurality of biological sequences based on the output distances and the time stamps associated with the plurality of biological sequences; and
a minimum spanning tree identifier (355) adapted to compute a minimum spanning tree, MST, of the generated graph and identify the computed MST as the phylogenetic tree.

8. A method of preparing a vaccine against a fast-evolving entity, based on a phylogenetic tree, comprising:

(i) selecting an individual isolate of the fast-evolving entity; and
(ii) preparing a vaccine based on the individual isolate selected in (i).

9. The method of claim 8, wherein the selecting of an individual isolate of the fast-evolving entity comprises the steps of:

(a) identifying the number of repeated amino acid mutations occurring in the isolate sequence; and/or
(b) identifying the position of the isolate in the phylogenetic tree; and
(c) selecting an isolate as a vaccine candidate when
the isolate sequence bears the maximum number of repeated amino acid mutations among all tested sequences which cluster in the phylogeny, occurred within a time span of 4 years before the season in which the vaccine is to be produced, and are located within the antigenic sites of the protein.

10. The method of claim 8 or 9, wherein the sequences are selected from RNA, DNA and protein sequences.

11. The method of any one of claims 8 to10, wherein the fast-evolving entity is a virus.

12. The method of claim 11, wherein the virus is an influenza virus.

13. The method of any of claims 8 to 12, wherein the phylogenetic tree is established using nucleic acid sequences encoding, or protein sequences of, a major surface protein, selected from hemagglutinin, HA, or neuraminidase, NA.

14. Vaccine produced by a method of any one of claims 8 to 13.

15. Method of selecting an influenza vaccine candidate, comprising:

(a) identifying a phylogenetic tree according to a method of any one of claims 1-7 from a plurality of biological sequences from influenza isolates
(b) identifying the number of repeated amino acid mutations occurring in each isolate sequence; and
(c) selecting an isolate as a vaccine candidate when

(c1) the isolate sequence bears the maximum number of repeated amino acid mutations that occurred within a time-span of 4 years before the season in which the vaccine is to be produced,
(c2) the isolate sequence bears the maximum number of repeated amino acid mutations that are located in antigenic sites and occurred within a time-span of 4 years before the season in which the vaccine is to be produced,
(c3) the isolate sequence bears the maximum number of repeated amino acid mutations that cluster in the phylogeny, are located in antigenic sites and occurred within a time-span of 3 years before the season in which the vaccine is to be produced, or
(c4) the isolate sequence bears the maximum number of repeated amino acid mutations that cluster in the phylogeny, are located in sites determined experimentally to change the viral phenotype and occurred within a time-span of 4 years before the season in which the vaccine is to be produced.

```
                    ┌─────────────────┐  ╭─100
                    │   Phylogeny     │
                    └─────────────────┘
                             │
                             ▼
        ┌──────────────────────────────────────┐  ╭─105
        │      collect dated sequence data      │
        └──────────────────────────────────────┘
                             │
                             ▼
        ┌──────────────────────────────────────┐  ╭─110
        │            align sequences            │
        └──────────────────────────────────────┘
                             │
                             ▼
        ┌──────────────────────────────────────┐  ╭─115
        │        compute distance matrix        │
        └──────────────────────────────────────┘
                             │
                             ▼
        ┌──────────────────────────────────────┐  ╭─120
        │ generate graph representation based on sampling
        │     dates of sequences and distances  │
        └──────────────────────────────────────┘
                             │
                             ▼
        ┌──────────────────────────────────────┐  ╭─125
        │   identify minimum spanning tree of graph
        └──────────────────────────────────────┘
                             │
                             ▼
                    ┌─────────────────┐
                    │     Return      │
                    └─────────────────┘
```

FIG. 1

```
        ╭─────────────────────────╮ ~200
        │   Graph Representation   │
        ╰─────────────────────────╯
                     │
                     ▼
   ┌──────────────────────────────────────────┐ ~202
   │              select sequence              │
   └──────────────────────────────────────────┘
                     │
                     ▼
   ┌──────────────────────────────────────────┐ ~204
   │  determine sampling date of selected sequence │◄───┐
   └──────────────────────────────────────────┘        │
                     │                                  │
                     ▼                                  │
   ┌──────────────────────────────────────────┐ ~206   │
   │  create node representing the selected sequence │  │
   └──────────────────────────────────────────┘        │
                     │                                  │
                     ▼                                  │
   ┌──────────────────────────────────────────┐ ~208   │
   │  associate created node with determined sampling date │ │
   └──────────────────────────────────────────┘        │
                     │                                  │
                     ▼                                  │
   ┌──────────────────────────────────────────┐ ~210   │
   │             mark selected sequence         │        │
   └──────────────────────────────────────────┘        │
       212           │                                  │
         ╲           ▼           214 ╲   ┌──────────────┐│
          ◇ Any unmarked ─────────────► │ select unmarked││
          ◇   sequence?     yes         │   sequence   │─┘
                     │ no               └──────────────┘
                     ▼
   ┌──────────────────────────────────────────┐ ~216
   │  determine node associated with earliest sampling date │
   └──────────────────────────────────────────┘
       218           │
         ╲           ▼
          ◇ Determination ─────────►╭──────────╮
          ◇    unique?      no      │  Return  │
                     │ yes          ╰──────────╯
                     ▼
   ┌──────────────────────────────────────────┐ ~220
   │     select node different from determined node │
   └──────────────────────────────────────────┘
    222 ╲            │
   ┌──────────────────────────────────────────┐   (B)
   │ add directed edge from determined node to selected node │◄─
   └──────────────────────────────────────────┘
                     │                              ~224
                     ▼
   ┌──────────────────────────────────────────┐
   │   weight edge with distance between sequence │
   │  represented by the determined node and sequence │
   │         represented by selected node       │
   └──────────────────────────────────────────┘
                     │
                     ▼
                    (A)
```

FIG. 2a

(A)

mark selected node as considered with
respect to determined node — 226

228
Any unconsidered
nodes? — 230 → select node different
from the determined
node from the
uncosidered nodes → (B)
yes

no

232 — mark determined node as done

234 — select node from undone nodes as first node ← (E)

determine sampling date associated with first node — 236

select node different from first node as second node — 238

240 — determine sampling date associated with second node ← (D)

242
Sampling
date of first node
earlier than sampling date
of second node? → (C)
no

yes

add directed edge from first node to second node — 244

weight edge with distance between sequence
represented by first node and sequence
represented by second node — 246

(C)

FIG. 2b

C

mark second node as considered with respect to first node —248

250
Any unconsidered nodes?

yes → 252 select node different from the first node from group of unconsidered nodes as second node → D

no

254 — mark second node as done

256
Any undone nodes?

no → Return

yes ↓

E

FIG. 2c

FIG. 3

EP 2 189 919 A1

FIG.4a

N=northern hemisphere isolate
S=southern hemisphere isolate
T=tropical isolate

FIG. 4b

antigenic signature clade

FIG.4c

FIG.5

FIG.6

Europäisches
Patentamt

European
Patent Office

Office européen
des brevets

## PARTIAL EUROPEAN SEARCH REPORT

**Application Number**

which under Rule 63 of the European Patent Convention EP 08 02 0435
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HOLLICH VOLKER ET AL: "Assessment of protein distance measures and tree-building methods for phylogenetic tree reconstruction" MOLECULAR BIOLOGY AND EVOLUTION, vol. 22, no. 11, November 2005 (2005-11), pages 2257-2264, XP002525184 ISSN: 0737-4038 * abstract * * page 2257, left-hand column, paragraph 1 - page 2258, right-hand column, paragraph 1 * * page 2262, right-hand column, paragraph 3 - page 2263, left-hand column, last paragraph * <br><br>-----<br><br>-/-- | 1-7,15 | INV.<br>G06F19/00<br>A61K39/145 |

|  |
|---|
| TECHNICAL FIELDS SEARCHED (IPC) |
| G06F<br>A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 23 July 2009 | Lüdemann, Susanna |

EPO FORM 1503 03.82 (P04E07)

**PARTIAL EUROPEAN SEARCH REPORT**

Application Number

EP 08 02 0435

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | GUINDON STEPHANE ET AL: "A simple, fast, and accurate algorithm to estimate large phylogenies by maximum likelihood." SYSTEMATIC BIOLOGY, vol. 52, no. 5, October 2003 (2003-10), pages 696-704, XP002525185 ISSN: 1063-5157 * abstract * * page 696, left-hand column, paragraph 1 - page 697, right-hand column, last paragraph * * page 699, right-hand column, paragraph 3 - page 700, left-hand column, paragraph 2 * * page 703, right-hand column, paragraph 2 - last paragraph * ----- | 1-7,15 | |
| X | HUERTA-CEPAS JAIME ET AL: "PhylomeDB: a database for genome-wide collections of gene phylogenies" NUCLEIC ACIDS RESEARCH, vol. 36, no. Sp. Iss. SI, January 2008 (2008-01), pages D491-D496, XP002525186 ISSN: 0305-1048 * abstract * * page D491, left-hand column, paragraph 1 - page D493, left-hand column, paragraph 1 * * figures 2,3 * ----- | 1-7,15 | TECHNICAL FIELDS SEARCHED (IPC) |
| | -/-- | | |

EPO FORM 1503 03.82 (P04C10)

Europäisches
Patentamt

European
Patent Office

Office européen
des brevets

**PARTIAL EUROPEAN SEARCH REPORT**

Application Number

EP 08 02 0435

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | RONQUIST FREDRIK ET AL: "MrBayes 3: Bayesian phylogenetic inference under mixed models." BIOINFORMATICS (OXFORD), vol. 19, no. 12, 12 August 2003 (2003-08-12), pages 1572-1574, XP002525187 ISSN: 1367-4803 * abstract * * page 1572, left-hand column, paragraph 1 - page 1573, right-hand column, paragraph 3 * | 1-7,15 | |
| | ----- | | |
| X | WOLF YURI I ET AL: "Long intervals of stasis punctuated by bursts of positive selection in the seasonal evolution of influenza A virus" BIOLOGY DIRECT, vol. 1, October 2006 (2006-10), XP002537401 * whole doc, in particular abstract and p. 11, right col., last 2 paragraphs * | 8-13 | TECHNICAL FIELDS SEARCHED (IPC) |
| | ----- | | |
| X | SMITH DEREK J ET AL: "Mapping the antigenic and genetic evolution of influenza virus" SCIENCE (WASHINGTON D C), vol. 305, no. 5682, 16 July 2004 (2004-07-16), pages 371-376, XP002537402 ISSN: 0036-8075 | 8 | |
| A | * the whole document * | 9-13 | |
| | ----- | | |
| E | WO 2009/051797 A1 (PEPTIMMUNE INC [US]; BONNIN DUSTAN [US]; ZANELLI ERIC [US]; KRIEGER JE) 23 April 2009 (2009-04-23) * the whole document * | 8-13 | |
| | ----- | | |
| | -/-- | | |

EPO FORM 1503 03.82 (P04C10)

Europäisches
Patentamt

European
Patent Office

Office européen
des brevets

**PARTIAL EUROPEAN SEARCH REPORT**

Application Number

EP 08 02 0435

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | WO 2006/086188 A2 (UNIV JOHNS HOPKINS [US]; RAY STUART C [US]; COX ANDREA L [US]; THOMAS) 17 August 2006 (2006-08-17) | 8 | |
| A | * whole document, in particular see [0028], [0029], [0046], claims and fig.'s 6-8). * | 9-13 | |
| | ----- | | |
| X | RILEY J P ET AL: "Distribution of linear antigenic epitopes on GP120 encoded in sibling clones of novel New York HIV-1 subtype B isolates." CELLULAR AND MOLECULAR BIOLOGY (NOISY-LE-GRAND, FRANCE) 1995, vol. 41 Suppl 1, 1995, pages S83-S91, XP009119980 ISSN: 0145-5680 | 8 | **TECHNICAL FIELDS SEARCHED** (IPC) |
| A | * whole document, in particular abstract, p. S85, S86, S89, left. col. 2nd paragraph. * | 9-13 | |
| | ----- | | |

Europäisches
Patentamt
European
Patent Office
Office européen
des brevets

**INCOMPLETE SEARCH
SHEET C**

Claim(s) completely searchable:
        1-13, 15

Claim(s) not searched:
        14

Reason for the limitation of the search:

The present claim 14 encompasses compounds defined only by their desired function  ("vaccines produced by the methods of claims 8-13), contrary to the requirement of clarity in Article 84 EPC, because the "result-to-be-achieved" type of definition does not allow the scope of the claim to be ascertained. The fact that any compound could be screened/produced does not overcome this objection, as the skilled person would not know beforehand whether it fell within the scope claimed. Undue experimentation would be required to screen/produce compounds randomly. Non-compliance with the substantive provisions is such that no meaningful search of claim 14 could be carried out at all (Rule 63 EPC).

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the requirements of unity of invention and relates to several inventions or groups of inventions, namely:

    1. claims: 1-7, 15

        Method and system for identifying a phylogenetic tree for a
        plurality of biological sequences.
                        ---

    2. claims: 8-14

        Method of preparing a vaccine against a fast-evolving
        entity.
                        ---

## ANNEX TO THE EUROPEAN SEARCH REPORT
## ON EUROPEAN PATENT APPLICATION NO.

EP 08 02 0435

This annex lists the patent family members relating to the patent documents cited in  the above-mentioned European search report.
The members are as contained in the European Patent Office EDP file on
The European Patent Office is in no way liable for these particulars which are merely given for the purpose of information.

23-07-2009

| Patent document cited in search report | | Publication date | Patent family member(s) | | Publication date |
|---|---|---|---|---|---|
| WO 2009051797 | A1 | 23-04-2009 | NONE | | |
| WO 2006086188 | A2 | 17-08-2006 | EP | 1846439 A2 | 24-10-2007 |

EPO FORM P0459

## REFERENCES CITED IN THE DESCRIPTION

### Non-patent literature cited in the description

- **S. Khumar et al.** MEGA3: Integrated software for Molecular Evolutionary Genetics Analysis and sequence alignment. *BRIEFINGS IN BIOINFORMATICS,* vol. 5 (2), 150-163
- **Swofford, D.L.** PAUP: Phylogenetic Analysis Using Parsimony (*and Other Methods''). Sinauer Associates, Inc. Publishers
- **Zwickl, D. J.** Genetic algorithm approaches for the phylogenetic analysis of large biological sequence datasets under the maximum likelihood criterion. *Ph.D. dissertation*
- **Guindon ; Olivier Gascuel.** A simple, fast and accurate method to estimate large phylogenies by maximum-likelihood. *SYSTEM BIOLOGY,* vol. 52, 696-704
- **Drummond AJ ; Rambaut A.** BEAST: Bayesian evolutionary analysis by sampling trees. *BMC EVOLUTIONARY BIOLOGY,* vol. 7, 214
- **Higgins, D.G. ; Bleasby, A.J. ; Fuchs, R.** CLUSTAL V: improved software for multiple sequence alignment. *Computer Applications in the Biosciences,* 1992, vol. 8 (2), 189-191
- **States et al.** *METHODS - A companion to Methods in Enzymology,* 1991, vol. 3, 66-70
- **R.E. Tarjan.** *Networks,* vol. 7, 25-35
- **F. J. Poelwijk et al.** *Nature,* 2007, vol. 445 (7126), 383
- **R. M. Bush et al.** *Mol. Biol. Evol.,* 1999, vol. 16 (11), 1457
- **Y. ; Hochberg Benjamini, Y.** *Journal of the Royal Statistical Society,* 1995, vol. 57 (289
- **D. C. Wiley et al.** *Nature,* 1981, vol. 289 (5796), 373
- **C. A. Smith et al.** *J Exp Med,* 1991, vol. 173 (4), 953
- **Gupta, V. et al.** *Vaccine,* 2006, vol. 24 (18), 3881-8
- **Wilson ; Cox.** Structural Basis of Immune Recognition of Influenza Virus Hemagglutinin. *Annual Review of Immunology,* 1990, vol. 8, 737-771